# EUROPEAN PATENT SPECIFICATION

(11) **EP 4 225 736 B1**
(45) Date of publication and mention of the grant of the patent: **24.07.2024**
(21) Application number: 21786502.1
(22) Date of filing: 08.10.2021
(51) Int. Cl.: C07D 213/70, C07D 213/79, C07D 213/81, C07D 213/84, C07D 409/04

(54) **PROCESS FOR THE PREPARATION OF 5-(1-CYANOCYCLOPROPYL)-PYRIDINE-2-CARBOXYLIC ACID ESTERS AMIDES AND NITRILES**
VERFAHREN ZUR HERSTELLUNG VON 5-(1-CYANOCYCLOPROPYL)-PYRIDINE-2-CARBONSÄUREESTER-AMIDEN UND -NITRILEN
PROCÉDÉ DE PRÉPARATION D'ESTERS DE L'ACIDE 5-(1-CYANOCYCLOPROPYL)-PYRIDINE-2-CARBOXYLIQUE AMIDES ET DE NITRILES

(30) Priority: 09.10.2020 EP 20201119
(43) Date of publication of application: 16.08.2023
(73) Proprietor: Syngenta Crop Protection AG, 4058 Basel (CH)
(72) Inventor: DUMEUNIER, Raphael, 4332 Stein (CH); BEAUDEGNIES, Renaud, 4332 Stein (CH); POULIOT, Martin, 4332 Stein (CH); GASPERS, Alexander, 4332 Stein (CH)
(86) International application number: PCT/EP2021/077899
(87) International publication number: WO 2022/074214

(56) References cited:
- WO-A1-2018/108726
- WO-A1-2019/234158
- JP-A- 2018 012 664

## Description

The present invention relates to the preparation of 5-(1-cyanocyclopropyl)-pyridine-2-carboxylic acid esters amides and nitriles that are useful as intermediates for the preparation of agrochemicals.

Certain 5-(1-cyanocyclopropyl)-pyridine-2-carboxylic acids with 3-alkylsulfanyl substituents (and certain corresponding esters, amides and salts thereof) are useful intermediates for the preparation of biologically active compounds in the agrochemical industries as previously described, for example, in: WO16087265, WO16121997, WO2019059244, WO2020071304, WO2020090585 and JP2020079325.

Known synthesis of 5-(1-cyanocyclopropyl)-pyridine-2-carboxylic acids with 3-alkylsulfanyl substituents involve many reaction steps. For example, a route to access the 3-ethylsulfanyl derivative have been reported from methyl 5-bromo-3-ethylsulfanyl-pyridine-2-carboxylate (in WO 2010/077565) and is shown in Scheme 1

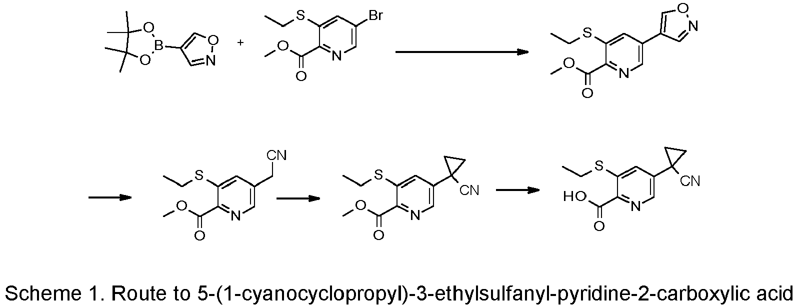

Two routes to access alkyl 5-bromo-3-ethylsulfanyl-pyridine-2-carboxylate have been reported (route A: CN105218437; route B: US2012/0165338 or J. Org. Chem. 2009, 74, 4547-4553) and are shown in Scheme 2 (R₁ is H, C₁-C₄alkyl, or an alkali metal ion)

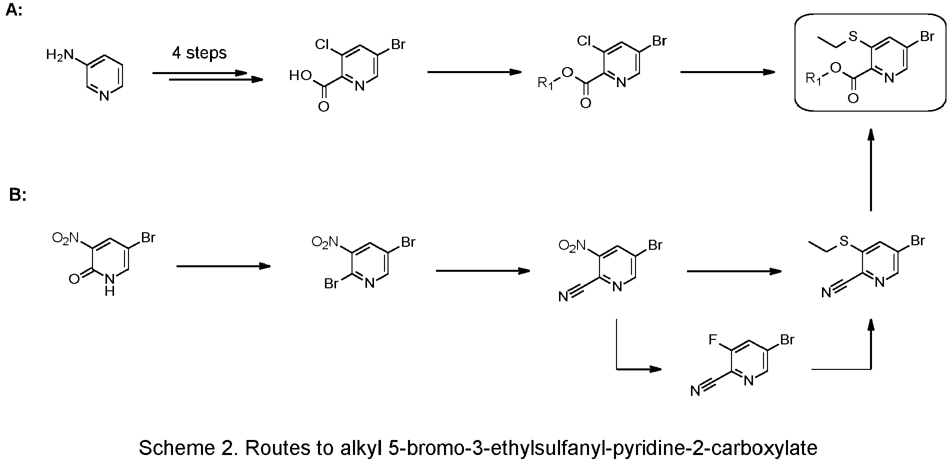

Such long and laborious syntheses are not suitable for preparing large amount of material due to low overall yields and large amount of waste generated. Therefore, it would be advantageous to have available a more efficient and economical route to these useful intermediates.

The present invention therefore provides a process for the preparation of 5-(1-cyanocyclopropyl)-pyridine-2-carboxylic acids esters amides and nitriles of formula I or agrochemically acceptable salts thereof 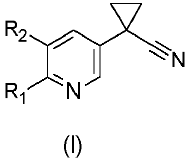 wherein R₁ is -CO₂R₄, -CO(NR₅R₆), carboxylate or cyano; R₂ is hydrogen, halogen or -SR₃; R₃ is C₁-C₄alkyl or C₃-C₆cycloalkyl-C₁-C₄alkyl; R₄ is hydrogen, -Si(CH₃)₃ or C₁-C₆alkyl; and R₅ and R₆ are, independently from each other, hydrogen or C₁-C₄alkyl.

In accordance with a first aspect of the present invention, the direct replacement of a halogen atom in 5-position of pyridines of formula (III), using a base and a reagent of formula (IV) yields a compound of formula (I) wherein R₁ and R₂ are defined as previously and Hal is halogen (Scheme 3).

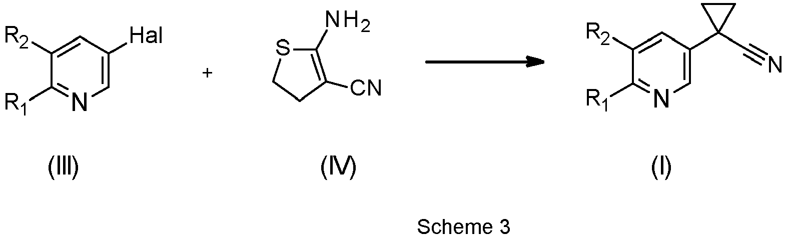

The compound of formula (IV) is known, commercially available and its synthesis described by Yamagata, Kenji et al in Chemical & Pharmaceutical Bulletin, 30(12), 4396-401; 1982 ; or by Wamhoff, Heinrich and Thiemig, Heinz Albrecht in Chemische Berichte, 118(11), 4473-85; 1985**;** or by Caspari, Philip et al in RSC Advances, 6(100), 98059-98065; 2016**.**

Thus, according to another aspect of the present invention, there is provided a process for the preparation of compounds of formula I (Scheme 4): 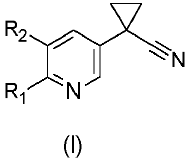
wherein R₁ is -CO₂R₄, -CO(NR₅R₆), carboxylate or cyano; R₂ is hydrogen, halogen or -SR₃; R₃ is C₁-C₄alkyl or C₃-C₆cycloalkyl-C₁-C₄alkyl; R₄ is hydrogen, -Si(CH₃)₃ or C₁-C₆alkyl; and R₅ and R₆ are, independently from each other, hydrogen or C₁-C₄alkyl, or an agrochemically acceptable salt of a compound of formula I; which process comprises:
   reacting a compound of formula (III) 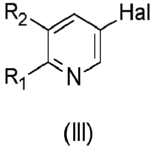
wherein R₁ and R₂ are defined as under formula I above and Hal is halogen
   with a compound of formula (IV), 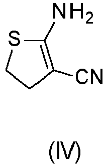
in the presence of a suitable base, in an appropriate solvent (or diluent);
to produce a compound of formula (I) or an agrochemically acceptable salt thereof.

Another aspect of the present invention provides certain compounds of formula I represented by the compounds of formula Ia 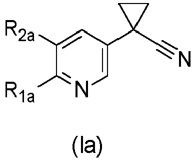
wherein R₁ₐ is -CO₂R₄ₐ, -CO(NR₅ₐR₆ₐ), carboxylate or cyano; R₂ₐ is hydrogen, halogen or -SR₃ₐ; R₃ₐ is C₁-C₄alkyl or C₃-C₆cycloalkyl-C₁-C₄alkyl; R₄ₐ is hydrogen, -Si(CH₃)₃ or C₁-C₆alkyl; and R₅ₐ and R₆ₐ are, independently from each other, hydrogen or C₁-C₄alkyl, or an agrochemically acceptable salt of a compound of formula la;
with the proviso that the compound of formula Ia is not: selected from the group consisting of a compound of the formula Ib, Ic and Id; 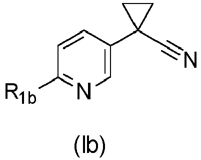
wherein R_{1b} is CN or-CO₂Me; 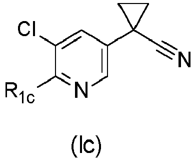
wherein R_{1c} is -CO₂H, -CO₂Me, -CO₂Et or -CONHMe; and 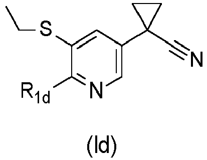
wherein R_{1d} is -CO₂H, -CO₂Me or -CONHMe.

The process of the present invention is demonstrated to be of great usefulness as it allows the synthesis of key building blocks for the preparation of agrochemicals in higher yields and with more favorable conditions with respect to previously described routes.

Compounds of formula I that are prepared by the inventive process, or compounds of formula III which are used in the inventive process, that have at least one basic centre can form, for example, acid addition salts, for example with strong inorganic acids such as mineral acids, for example perchloric acid, sulfuric acid, nitric acid, nitrous acid, a phosphorus acid or a hydrohalic acid, with strong organic carboxylic acids, such as C₁-C₄alkanecarboxylic acids which are unsubstituted or substituted, for example by halogen, for example acetic acid, such as saturated or unsaturated dicarboxylic acids, for example oxalic acid, malonic acid, succinic acid, maleic acid, fumaric acid or phthalic acid, such as hydroxycarboxylic acids, for example ascorbic acid, lactic acid, malic acid, tartaric acid or citric acid, or such as benzoic acid, or with organic sulfonic acids, such as C₁-C₄alkane- or arylsulfonic acids which are unsubstituted or substituted, for example by halogen, for example methane- or p-toluenesulfonic acid. Compounds of formula I, or compounds of formula III which are used in the inventive process, which have at least one acidic group can form, for example, salts with bases, for example mineral salts such as alkali metal or alkaline earth metal salts, for example sodium, potassium, lithium or magnesium salts, or salts with ammonia or an organic amine, such as morpholine, piperidine, pyrrolidine, a mono-, di- or tri-lower-alkylamine, for example ethyl-, diethyl-, triethyl- or dimethylpropylamine, or a mono-, di- or trihydroxy-lower-alkylamine, for example mono-, di- or triethanolamine. In a preferred embodiment, compounds of formula III which are used in the inventive process are either neutral or are negatively charged, particularly when the substituent R₁ is a carboxylate anion.

In each case, the compounds of formula (I) that are prepared by the process according to the invention are in free form or in salt form, e.g. an agronomically usable salt form.

The term "C₁-Cₙalkyl" as used herein refers to a saturated straight-chain or branched hydrocarbon radical attached via any of the carbon atoms having 1 to n carbon atoms, for example, any one of the radicals methyl, ethyl, n-propyl, 1-methylbutyl, 2-methylbutyl, 3-methylbutyl, 2, 2-dimethylpropyl, 1-ethylpropyl, n-hexyl, n-pentyl, 1, 1-dimethylpropyl, 1, 2-dimethylpropyl, 1- methylpentyl, 2-methylpentyl, 3-methylpentyl, 4-methylpentyl, 1, 1-dimethylbutyl, 1,2- dimethylbutyl, 1,3-dimethylbutyl, 2, 2-dimethylbutyl, 2, 3-dimethylbutyl, 3, 3-dimethylbutyl, 1-ethylbutyl, 2-ethylbutyl, 1,1, 2-trimethylpropyl, 1,2, 2-trimethylpropyl, 1-ethyl-1- methylpropyl, or 1-ethyl-2-methylpropyl.

The term "C₃-C₆cycloalkyl" as used herein refers to 3-6 membered cycloalkyl groups such as cyclopropane, cyclobutane, cyclopentane and cyclohexane.

The suffix "-C₁-C₄alkyl" after terms such as "C₃-C₆cycloalkyl" as used herein refers to a straight chain or branched saturated alkyl radical which is substituted by C₃-C₆cycloalkyl. An example of C₃-C₆cycloalkyl-C₁-C₄alkyl is for example, cyclopropylmethyl.

Halogen is generally fluorine, chlorine, bromine or iodine.

Additional aspects of the process according to the invention for making compounds of formula (I) are further detailed and explained by reference to scheme 5, wherein R₁, R₂ and Hal are defined as above. Some intermediates compounds are made and further transformed in the process of making compounds of formula (I), which can then be detailed as passing through the following intermediates in a single pot operation (Scheme 5)

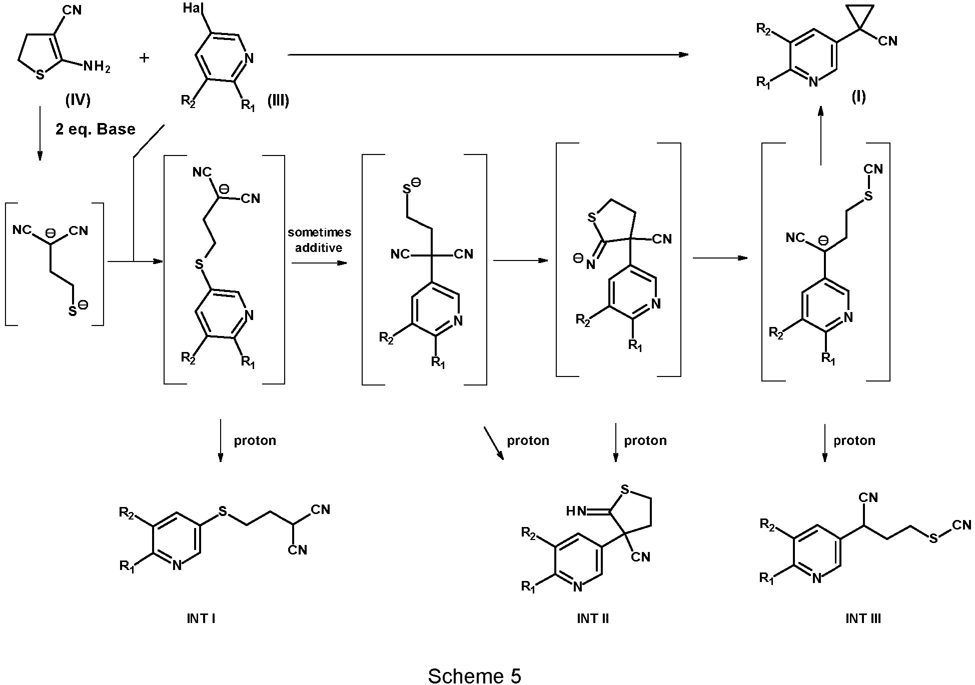

The intermediate salts can sometimes be protonated, isolated and characterized under the forms of INT I, INT II, or INT III before their complete transformations into compounds of formula (I). In this regard, the compounds INT I, INT II, or INT III can also be resubmitted to basic conditions in a separate, subsequent step to provide compounds of formula (I).In the process according to the invention for making compounds of formula (I), examples of suitable bases are alkali metal hexamethyldisilazides, alkaline earth metal hexamethyldisilazides, alkali metal hydroxides, alkali metal alkoxides or alkaline earth metal alkoxides. Examples which may be mentioned are sodium hydroxide, potassium hydroxide, sodium methanolate, sodium tertiobutanolate, and potassium tertiobutanolate; preferably an alkali metal hydroxide, more preferably sodium hydroxide. In one embodiment, for intermediate I (INT I), a special case of R₁ being carboxylate anion requires the cations to partly or fully consist of magnesium or zinc cations for the final steps of the reaction to proceed (such as those shown in scheme 5). Such a base as magnesium bis(tertio-butanolate) or magnesium bis(hexamethyldisilazide) can then be used from the start without any additives being necessary. Alternatively, bases such as sodium or potassium hydroxides or alkoxides can be used when followed by the addition of magnesium^{(II)}, zinc^{(II)} or Al^{(III)} salts as additives, such as for example MgCl₂, ZnCl₂, Al(OtBu)₃ or AlCl₃ (referred to in scheme 5 as "sometimes additive").

Such additives can be introduced into the reaction after the formation of INT I or, in some cases, from the start of the reaction. An example of the latter is the concomitant use of NaOtBu (as base) and Al(OtBu)₃ (as additive) from the start of the reaction. The water or the alcohol generated from the base, after generation of INT I during the reaction, can advantageously be removed from the reaction mixture by distillation before the introduction of the additive. This can be done without solvent removal if the solvent used has high boiling point (such as NMP or DMSO), or with removal of only a fraction of the solvent if the boiling point comes closer to the alcohol or water (DMF). Removing the water or the alcohol from the reaction mixture before introduction of the additive allows the use of reduced amounts of the latter. In a preferred embodiment, the reaction will also proceed without removal (continuous or after INT I formation) of water or alcohol provided that sufficient equivalents of additive are introduced.

The following Table X is intended to illustrate some combinations of bases, solvents and/or additives within the scope of the present invention. Preferably, if an additive is used, it is introduced into the reaction after the formation of INT I.

**TABLE X**

| Entry | Base | Solvent | Additive | Temp | Yield of Compoud (I) |
|---|---|---|---|---|---|
| 1 | Solid NaOMe | NMP | MgCl₂ | 65°C | 87% |
| 2 | Solid NaOtBu | NMP | MgCl₂ | 65°C | 90% (example 11) |
| 3 | NaOH powder | DMF | MgCl₂ | 65°C | 91% |
| 4 | NaOH powder | DMSO | ZnCl₂ | 65°C | *circa* 70% |
| 5 | Mg(OtBu)₂ | DMF | - | 65°C | 58% |
| 6 | Mg(HMDS)₂ | DMF | - | 65°C | 72% |
| 7 | LiHMDS | DMF | Al(OtBu)₃ | 65°C | 49% |
| 8 | Solid NaOtBu | NMP | AlCl₃ | 65°C | *circa* 70% |

A preferred embodiment of the process for the preparation of compounds of formula I as shown and explained in scheme 5 is further detailed by reference to scheme 6. In particular, alternatives to the use of magnesium(II), zinc(II) or aluminium (III) additives for preparation of compounds of formula I by the inventive process are for example, the addition of trimethylsilylchloride (TMSCI) after distillation of the water or alcohol generated during the formation of INT I. This creates a silyl ester *in situ* from INT I (Si-INT I) which is activated enough to engage spontaneously into the further transformations to the next silylated intermediates Si-INT-II and Si-INT-III, all the way to a silylated ester analogue of a compound of formula (In scheme 6, a silylated ester analogue of a compound of formula I is represented by a a silylated ester analogue of a compound of formula I-1). The latter is cleaved spontaneously during the aqueous work-up of the reaction to finally deliver compound I-1, as depicted in Scheme 6:

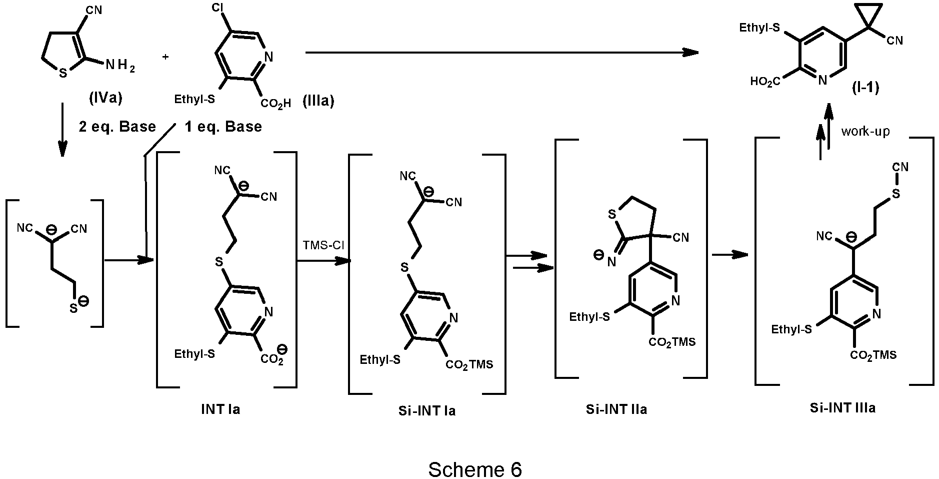

In the process according to the invention of making compounds of formula (I), appropriate solvents (or diluents) are polar solvents, such as dimethylformamide, dimethylsulfoxide, N-methyl-pyrrolidine, dimethylacetamide, sulfolane, N,N'-dimethylpropyleneurea (DMPU); more preferably polar organic solvents chosen from dimethylformamide, dimethylsulfoxide, N-methyl-pyrrolidine.

In one embodiment, in the process according to the invention of making compounds of formula (I), the reaction is advantageously carried out in a temperature range from approximately 0°C to approximately +100°C, preferably from approximately +20°C to approximately +80°C.

Another preferred embodiment of the process for the preparation of compounds of formula I as shown and explained in scheme 5 is further detailed by reference to scheme 7 which shows specific embodiments of the formulae I, III, INT 1, INT II and INT III as represented by compounds of formulae I-1, IIIa, INT la, INT IIa and INT IIIa, respectively.

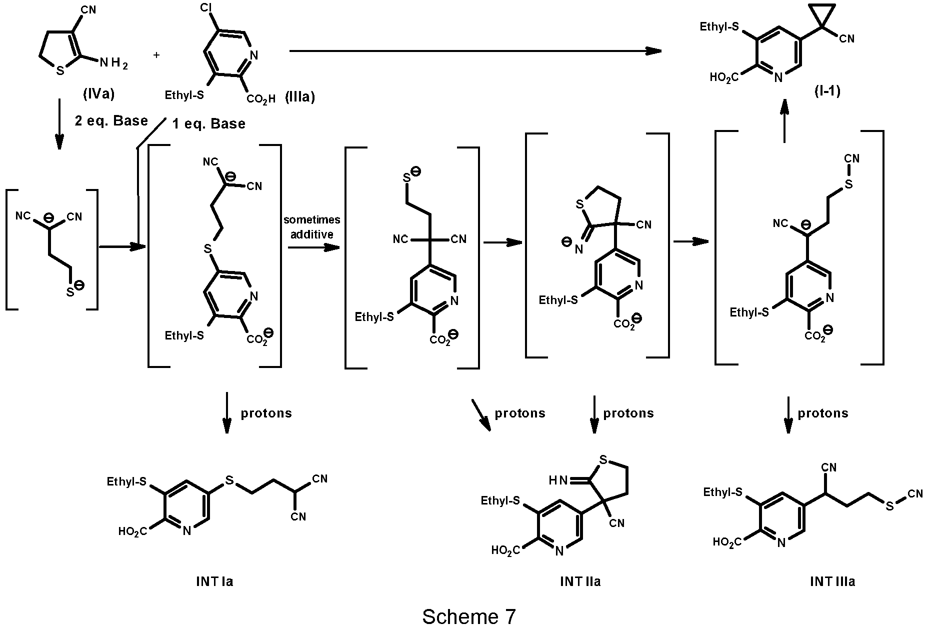

In one embodment, the anions of INT I are intermediates which often do accumulate to a reasonable extent during the course of the reaction, so that if the reaction is interrupted before completion, INT I can be isolated and purified, as can be seen in the preparatory examples. In particular, if the first steps to anions of INT I are run at lower temperature (room temperature or even -10°C to -20°C), a good yield of INT I can be achieved, if it is wished to interrupt the reaction at this stage. A special case is INT Ia (with R₁ being carboxylic acid), as the anions of the latter will accumulate completely as long as no cations such as Mg(II), Zn(ll) or AI(III) are present in the reaction mixture. An excellent yield in isolating INT Ia can then be achieved if desired (see example 18).

In another embodiment, anions of INT II and INT III do not accumulate to significant levels in the reaction mixture. In some cases, they can be observed by *in situ* monitoring or (their protonated forms) by LCMS of reactions samples, but their amounts are too low to be able to interrupt the reaction, isolate and characterize pure samples of INT II or INT III. For getting proper analysis and pure samples of those intermediates, a specific synthesis can be made to properly characterize them, for example by following the steps described in scheme 8 for getting INT IIa and INT IIIa

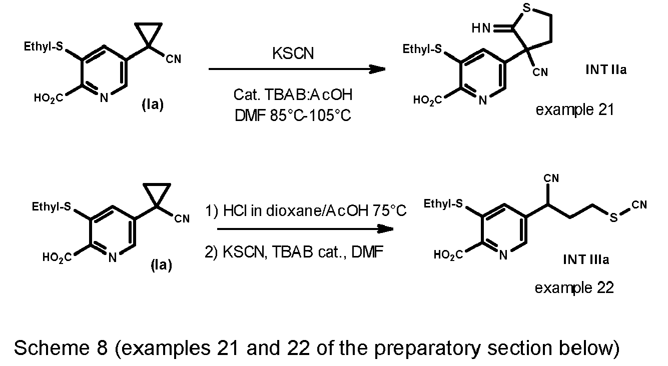

In another aspect, the present invention also relates to compounds of the formula INT I 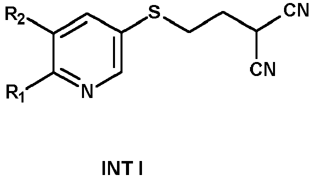 wherein R₁ and R₂ are defined as under formula I above, or an agrochemically acceptable salt of a compound of formula INT I.

In a preferred group of compounds of formula INT I, R₁ is -CO₂R₄, -CONH₂, carboxylate or cyano; wherein R₄ is hydrogen, -Si(CH₃)₃, methyl, ethyl, isopropyl, tert-butyl, or sec-butyl; and R₂ is hydrogen, halogen or -SR₃; wherein R₃ is ethyl or cyclopropyl methyl; preferably, R₂ is hydrogen, chloro or -SR₃; wherein R₃ is ethyl.

Another group of compounds according to the invention are those of the formula INT II 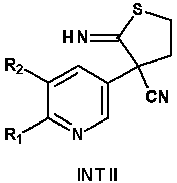 wherein R₁ and R₂ are defined as under formula I above, or an agrochemically acceptable salt of a compound of formula INT II.

In a preferred group of compounds of formula INT II, R₁ is -CO₂R₄, -CONH₂, carboxylate or cyano; wherein R₄ is hydrogen, -Si(CH₃)₃, methyl, ethyl, isopropyl, tert-butyl, or sec-butyl; and R₂ is hydrogen, halogen or -SR₃; wherein R₃ is ethyl or cyclopropyl methyl; preferably, R₂ is hydrogen, chloro or -SR₃; wherein R₃ is ethyl.

Another group of compounds according to the invention are those of the formula INT III 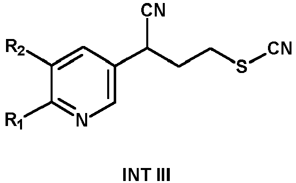 wherein R₁ and R₂ are defined as under formula I above, or an agrochemically acceptable salt of a compound of formula INT III.

In a preferred group of compounds of formula INT II, R₁ is -CO₂R₄, -CONH₂, carboxylate or cyano; wherein R₄ is hydrogen, -Si(CH₃)₃, methyl, ethyl, isopropyl, tert-butyl, or sec-butyl; and R₂ is hydrogen, halogen or -SR₃; wherein R₃ is ethyl or cyclopropyl methyl; preferably, R₂ is hydrogen, chloro or -SR₃; wherein R₃ is ethyl.

Certain preferred embodiments according to the invention are provided as set out below.

Embodiment 1 provides a process for the preparation of a compound of formula I by reacting a compound of formula III with a compound of formula IVa, as defined in scheme 3 above.

Embodiment 2 provides a process for the preparation of a compound of formula I by reacting a compound of formula III with a compound of formula IV, as defined in scheme 4 above.

Embodiment 3 provides a process for the preparation of a compound of formula I by reacting a compound of formula III with a compound of formula IV, as defined in scheme 5 above.

Embodiment 4 provides a process for the preparation of a compound of formula I by reacting a compound of formula IIIa with a compound of formula IVa, as defined in scheme 6 above.

Embodiment 5 provides a process for the preparation of a compound of formula I by reacting a compound of formula IIIa with a compound of formula IVa, as defined in scheme 7 above.

Embodiment 6 provides compounds of formula INT I, as defined above.

Embodiment 7 provides compounds of formula INT II, as defined above.

Embodiment 8 provides compounds of formula INT III, as defined above.

Embodiment 9 provides compounds of formula I represented by the compounds of formula la, as defined above.

With respect to embodiments 1 - 8, preferred values of R₁, R₂, R₃, R₄, R₅, R₆ and Hal are (as applicable), in any combination thereof, as set out below:
Preferably R₁ is -CO₂R₄, -CO(NR₅R₆) or cyano; wherein R₄ is hydrogen or C₁-C₆alkyl; and R₅ and R₆ are, independently from each other, hydrogen or C₁-C₄alkyl.

More preferably, R₁ is -CO₂R₄, -CO(NR₅R₆) or cyano; wherein R₄ is hydrogen or C₁-C₄alkyl; and R₅ and R₆ are, independently from each other, hydrogen or methyl.

Also preferred is when R₁ is -CO₂R₄, -CONH₂ or cyano; wherein R₄ is hydrogen, methyl, ethyl, isopropyl, tert-butyl, Si(CH₃)₃ or sec-butyl.

Further preferred is when R₁ is -COzH, -CONH₂ or a cyano group.

Most preferably R₁ is -CO₂H or, in case of a salt, a carboxylate anion thereof.

Preferably, agrochemically acceptable salts are alkali metal or alkaline earth metal salts.

More preferably, agrochemically acceptable salts are sodium, potassium, lithium or magnesium salts. Most preferably, agrochemically acceptable salts are sodium, magnesium or lithium.

Also preferred when R₁ is a carboxylate anion of -CO₂H, is that the corresponding cations partly or fully consist of magnesium.

Preferably, R₂ is hydrogen, halogen or -SR₃; wherein R₃ is C₁-C₄alkyl or C₃-C₆cycloalkyl-C₁-C₄alkyl; preferably, wherein R₃ is ethyl or cyclopropyl methyl.

More preferably, R₂ is hydrogen, halogen or -SR₃; wherein R₃ is ethyl or cyclopropyl methyl; preferably, R₂ is hydrogen, chloro or -SR₃; wherein R₃ is ethyl.

Most preferably, R₂ is -SR₃; wherein R₃ is ethyl.

Preferably, Hal is halogen.

More preferably, Hal is fluoro, chloro or bromo.

Also preferred is when Hal is chloro or bromo.

Most preferably, Hal is chloro.

With respect to embodiment 9, R₁ₐ and R₂ₐ are defined as under formula Ia above, and preferred values of R₁ₐ and R₂ₐ are as set out below.

Preferably when R₂ₐ is hydrogen, R₁ₐ is CO₂Et, CO₂iPr, CO₂sBu, CO₂tBu, CO₂H, CONH₂, or CO₂Si(CH₃)₃.

Preferably when R₂ₐ is chloro, R₁ₐ is CN, CO₂iPr, CO₂sBu, CO₂tBu, CONH₂ or CO₂Si(CH₃)₃. Preferably when R₂ₐ is -SEt, R₁ₐ is CN, CO₂Et, CO₂iPr, CO₂sBu, CO₂tBu, CONH₂ or CO₂Si(CH₃)₃. The examples which follow in Tables A, B, C, D and E below are intended to illustrate the invention and show preferred compounds of formulae I, III, INT I, INT II and INT III that can be used and/or prepared according to the process described above. In the tables below, "Me" represents methyl; "Et" represents ethyl; "iPr" represents isopropyl; "sBu" represents sec-butyl; "tBu" represents tert-butyl.

The following table A illustrates specific compounds of formula III:

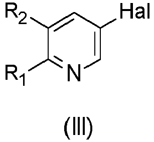

| TABLE A | | | |
|---|---|---|---|
| Compound of Formula III | R₁ | R₂ | Hal |
| 1 | CN | H | F |
| 2 | CO₂Me | H | F |
| 3 | CO₂Et | H | F |
| 4 | CO₂iPr | H | F |
| 5 | CO₂sBu | H | F |
| 6 | CO₂tBu | H | F |
| 7 | CO₂H | H | F |
| 8 | CONH₂ | H | F |
| 9 | CO₂Si(CH₃)₃ | H | F |
| 10 | CN | H | Cl |
| 11 | CO₂Me | H | Cl |
| 12 | CO₂Et | H | Cl |
| 13 | CO₂iPr | H | Cl |
| 14 | CO₂sBu | H | Cl |
| 15 | CO₂tBu | H | Cl |
| 16 | CO₂H | H | Cl |
| 17 | CONH₂ | H | Cl |
| 18 | CO₂Si(CH₃)₃ | H | Cl |
| 19 | CN | H | Br |
| 20 | CO₂Me | H | Br |
| 21 | CO₂Et | H | Br |
| 22 | CO₂iPr | H | Br |
| 23 | CO₂sBu | H | Br |
| 24 | CO₂tBu | H | Br |
| 25 | CO₂H | H | Br |
| 26 | CONH₂ | H | Br |
| 27 | CO₂Si(CH₃)₃ | H | Br |
| 28 | CN | SCH₂CH₃ | F |
| 29 | CO₂Me | SCH₂CH₃ | F |
| 30 | CO₂Et | SCH₂CH₃ | F |
| 31 | CO₂iPr | SCH₂CH₃ | F |
| 32 | CO₂sBu | SCH₂CH₃ | F |
| 33 | CO₂tBu | SCH₂CH₃ | F |
| 34 | CO₂H | SCH₂CH₃ | F |
| 35 | CONH₂ | SCH₂CH₃ | F |
| 36 | CO₂Si(CH₃)₃ | SCH₂CH₃ | F |
| 37 | CN | SCH₂CH₃ | Cl |
| 38 | CO₂Me | SCH₂CH₃ | Cl |
| 39 | CO₂Et | SCH₂CH₃ | Cl |
| 40 | CO₂iPr | SCH₂CH₃ | Cl |
| 41 | CO₂sBu | SCH₂CH₃ | Cl |
| 42 | CO₂tBu | SCH₂CH₃ | Cl |
| 43 | CO₂H | SCH₂CH₃ | Cl |
| 44 | CONH₂ | SCH₂CH₃ | Cl |
| 45 | CO₂Si(CH₃)₃ | SCH₂CH₃ | Cl |
| 46 | CN | SCH₂CH₃ | Br |
| 47 | CO₂Me | SCH₂CH₃ | Br |
| 48 | CO₂Et | SCH₂CH₃ | Br |
| 49 | CO₂iPr | SCH₂CH₃ | Br |
| 50 | CO₂sBu | SCH₂CH₃ | Br |
| 51 | CO₂tBu | SCH₂CH₃ | Br |
| 52 | CO₂H | SCH₂CH₃ | Br |
| 53 | CONH₂ | SCH₂CH₃ | Br |
| 54 | CO₂Si(CH₃)₃ | SCH₂CH₃ | Br |
| 55 | CN | Cl | F |
| 56 | CO₂Me | Cl | F |
| 57 | CO₂Et | Cl | F |
| 58 | CO₂iPr | Cl | F |
| 59 | CO₂sBu | Cl | F |
| 60 | CO₂tBu | Cl | F |
| 62 | CO₂H | Cl | F |
| 62 | CONH₂ | Cl | F |
| 63 | CO₂Si(CH₃)₃ | Cl | F |
| 64 | CN | Cl | Cl |
| 65 | CO₂Me | Cl | Cl |
| 66 | CO₂Et | Cl | Cl |
| 67 | CO₂iPr | Cl | Cl |
| 68 | CO₂sBu | Cl | Cl |
| 69 | CO₂tBu | Cl | Cl |
| 70 | CO₂H | Cl | Cl |
| 71 | CONH₂ | Cl | Cl |
| 72 | CO₂Si(CH₃)₃ | Cl | Cl |
| 73 | CN | Cl | Br |
| 74 | CO₂Me | Cl | Br |
| 75 | CO₂Et | Cl | Br |
| 76 | CO₂iPr | Cl | Br |
| 77 | CO₂sBu | Cl | Br |
| 78 | CO₂tBu | Cl | Br |
| 79 | CO₂H | Cl | Br |
| 80 | CONH₂ | Cl | Br |
| 81 | CO₂Si(CH₃)₃ | Cl | Br |

and the agrochemically acceptable salts of the compounds of formula III in Table A (and in the case of compounds where R₁ is -CO₂H, including the sodium, potassium, magnesium or lithium salts (i.e., wherein R₁ is a carboxylate anion)).

The following table B illustrates specific compounds of formula INT I:

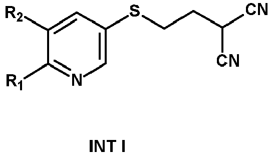

| TABLE B | | |
|---|---|---|
| Compound of formula INT I | R₁ | R₂ |
| 1 | CN | H |
| 2 | CO₂Me | H |
| 3 | CO₂Et | H |
| 4 | CO₂iPr | H |
| 5 | CO₂sBu | H |
| 6 | CO₂tBu | H |
| 7 | CO₂H | H |
| 8 | CONH₂ | H |
| 9 | CO₂Si(CH₃)₃ | H |
| 10 | CN | SCH₂CH₃ |
| 11 | CO₂Me | SCH₂CH₃ |
| 12 | CO₂Et | SCH₂CH₃ |
| 13 | CO₂iPr | SCH₂CH₃ |
| 14 | CO₂sBu | SCH₂CH₃ |
| 15 | CO₂tBu | SCH₂CH₃ |
| 16 | CO₂H | SCH₂CH₃ |
| 17 | CONH₂ | SCH₂CH₃ |
| 18 | CO₂Si(CH₃)₃ | SCH₂CH₃ |
| 19 | CN | Cl |
| 20 | CO₂Me | Cl |
| 21 | CO₂Et | Cl |
| 22 | CO₂iPr | Cl |
| 23 | CO₂sBu | Cl |
| 24 | CO₂tBu | Cl |
| 25 | CO₂H | Cl |
| 26 | CONH₂ | Cl |
| 27 | CO₂Si(CH₃)₃ | Cl |

and the agrochemically acceptable salts of the compounds of formula INT I in Table B (and in the case of compounds where R₁ is -CO₂H, including the sodium, potassium, magnesium or lithium salts (i.e., wherein R₁ is a carboxylate anion)).

The following table C illustrates specific compounds of formula INT II:

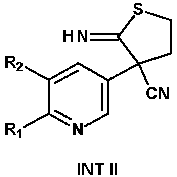

| TABLE C | | |
|---|---|---|
| Compound of Formula INT II | R₁ | R₂ |
| 1 | CN | H |
| 2 | CO₂Me | H |
| 3 | CO₂Et | H |
| 4 | CO₂iPr | H |
| 5 | CO₂sBu | H |
| 6 | CO₂tBu | H |
| 7 | CO₂H | H |
| 8 | CONH₂ | H |
| 9 | CO₂Si(CH₃)₃ | H |
| 10 | CN | SCH₂CH₃ |
| 11 | CO₂Me | SCH₂CH₃ |
| 12 | CO₂Et | SCH₂CH₃ |
| 13 | CO₂iPr | SCH₂CH₃ |
| 14 | CO₂sBu | SCH₂CH₃ |
| 15 | CO₂tBu | SCH₂CH₃ |
| 16 | CO₂H | SCH₂CH₃ |
| 17 | CONH₂ | SCH₂CH₃ |
| 18 | CO₂Si(CH₃)₃ | SCH₂CH₃ |
| 19 | CN | Cl |
| 20 | CO₂Me | Cl |
| 21 | CO₂Et | Cl |
| 22 | CO₂iPr | Cl |
| 23 | CO₂sBu | Cl |
| 24 | CO₂tBu | Cl |
| 25 | CO₂H | Cl |
| 26 | CONH₂ | Cl |
| 27 | CO₂Si(CH₃)₃ | Cl |

and the agrochemically acceptable salts of the compounds of formula INT II in Table C (and in the case of compounds where R₁ is -CO₂H, including the sodium, potassium, magnesium or lithium salts (i.e., wherein R₁ is a carboxylate anion)).

The following table D illustrates specific compounds of formula INT III:

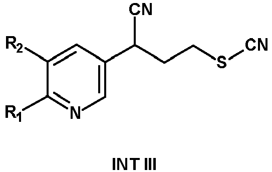

| TABLE D | | |
|---|---|---|
| Compound of Formula INT III | R₁ | R₂ |
| 1 | CN | H |
| 2 | CO₂Me | H |
| 3 | CO₂Et | H |
| 4 | CO₂iPr | H |
| 5 | CO₂sBu | H |
| 6 | CO₂tBu | H |
| 7 | CO₂H | H |
| 8 | CONH₂ | H |
| 9 | CO₂Si(CH₃)₃ | H |
| 10 | CN | SCH₂CH₃ |
| 11 | CO₂Me | SCH₂CH₃ |
| 12 | CO₂Et | SCH₂CH₃ |
| 13 | CO₂iPr | SCH₂CH₃ |
| 14 | CO₂sBu | SCH₂CH₃ |
| 15 | CO₂tBu | SCH₂CH₃ |
| 16 | CO₂H | SCH₂CH₃ |
| 17 | CONH₂ | SCH₂CH₃ |
| 18 | CO₂Si(CH₃)₃ | SCH₂CH₃ |
| 19 | CN | Cl |
| 20 | CO₂Me | Cl |
| 21 | CO₂Et | Cl |
| 22 | CO₂iPr | Cl |
| 23 | CO₂sBu | Cl |
| 24 | CO₂tBu | Cl |
| 25 | CO₂H | Cl |
| 26 | CONH₂ | Cl |
| 27 | CO₂Si(CH₃)₃ | Cl |

and the agrochemically acceptable salts of the compounds of formula INT III in Table D (and in the case of compounds where R₁ is -CO₂H, including the sodium, potassium, magnesium or lithium salts (i.e., wherein R₁ is a carboxylate anion)).

The following table E illustrates specific compounds of formula I:

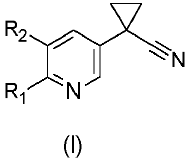

| TABLE E | | |
|---|---|---|
| Compound of formula I | R₁ | R₂ |
| 1 | CN | H |
| 2 | CO₂Me | H |
| 3 | CO₂Et | H |
| 4 | CO₂iPr | H |
| 5 | CO₂sBu | H |
| 6 | CO₂tBu | H |
| 7 | CO₂H | H |
| 8 | CONH₂ | H |
| 9 | CO₂Si(CH₃)₃ | H |
| 10 | CN | SCH₂CH₃ |
| 11 | CO₂Me | SCH₂CH₃ |
| 12 | CO₂Et | SCH₂CH₃ |
| 13 | CO₂iPr | SCH₂CH₃ |
| 14 | CO₂sBu | SCH₂CH₃ |
| 15 | CO₂tBu | SCH₂CH₃ |
| 16 | CO₂H | SCH₂CH₃ |
| 17 | CONH₂ | SCH₂CH₃ |
| 19 | CO₂Si(CH₃)₃ | SCH₂CH₃ |
| 19 | CN | Cl |
| 20 | CO₂Me | Cl |
| 21 | CO₂Et | Cl |
| 22 | CO₂iPr | Cl |
| 23 | CO₂sBu | Cl |
| 24 | CO₂tBu | Cl |
| 25 | CO₂H | Cl |
| 26 | CONH₂ | Cl |
| 27 | CO₂Si(CH₃)₃ | Cl |

and the agrochemically acceptable salts of the compounds of formula I in Table E (and in the case of compounds where R₁ is -CO₂H, including the sodium, potassium, magnesium or lithium salts (i.e., wherein R₁ is a carboxylate anion)).

### Preparatory examples:

Purity of starting materials, crudes and products was determined with quantitative ¹H NMR using 1,3,5-trimethoxy benzene as an internal standard.

### Example 1: Preparation of 5-(1-cyanocyclopropyl)pyridine-2-carbonitrile

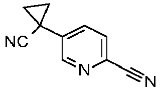

To a solution of 5-amino-2,3-dihydrothiophene-4-carbonitrile (0.1g, 0.79mmol) in DMF (3.2mL) was added 5-chloropyridine-2-carbonitrile (0.11 g, 0.79 mmol) and sodium tertio-butanolate (0.152g, 1.585 mmol). The reaction mixture was stirred at ambient temperature for 3 hours, then at 65°C for one hour more, before being allowed to cool down to room temperature. The reaction mixture was poured onto aqueous saturated ammonium chloride (10mL), acidified with 1M HCl (10mL) and extracted four times with dichloromethane (4x30mL). The combined organic layers were dried over solid anhydrous Magnesium sulfate, filtered and evaporated under reduced pressure. QNMR indicated a chemical yield of 78%. The crude was purified by column chromatography to obtain the title compound in 74% isolated yield (99.3mg). ¹H NMR (400 MHz, CDCl₃): δ (ppm) = 8.61 (dd, *J* = 2.6, 0.7 Hz, 1H), 7.80-7.74 (m, 1H), 7.73 - 7.65 (m, 1H), 1.99 - 1.91 (m, 2H), 1.60 - 1.53 (m, 2H)

### Example 2: Preparation of 5-(1-cyanocyclopropyl)-3-ethylsulfanyl-pyridine-2-carbonitrile

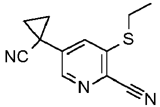

To a solution of 5-bromo-3-ethylsulfanyl-pyridine-2-carbonitrile (0.2g, 0.823mmol) and 5-amino-2,3-dihydrothiophene-4-carbonitrile (0.135g, 92.4% w/w purity, 0.987mmol) in DMF (1.64mL) was added dropwise a solution of Sodium bis(trimethylsilyl)amide (1M in THF, 1.6mL, 1.6mmol) at room temperature. The reaction mixture was stirred 1h before being quenched with aqueous saturated ammonium chloride (5mL) and extracted three times with ethyl acetate (3x20mL). The combined organic layers were washed with brine (20mL), dried over solid anhydrous Magnesium sulfate, filtered and evaporated under reduced pressure yielding a brownish oil (219.3mg). QNMR indicated a strength of 39.4% w/w, leading to a chemical yield of 46%.¹H NMR (400 MHz, d6-DMSO) δ ppm 1.26 - 1.32 (m, 3 H) 1.77 - 1.83 (m, 2H) 1.88 - 1.95 (m, 2 H) 3.15 - 3.26 (m, 2 H) 7.75 - 7.77 (d, *J*=2.20 Hz, 1 H) 8.50 (d, *J*=2.20 Hz, 1 H) LC-MS: ret.: 0.90 min, m/z +H⁺: 230

### Example 3: Preparation of 3-chloro-5-(1-cyanocyclopropyl)pyridine-2-carbonitrile

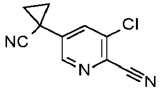

To a solution of 3,5-dichloropyridine-2-carbonitrile (0.3g, 1.734mmol) and 5-amino-2,3-dihydrothiophene-4-carbonitrile (0.255g, 94.3% w/w purity, 1.907mmol) in DMF (2.6mL) was added dropwise a solution of Sodium bis(trimethylsilyl)amide (1M in THF, 3.8mL, 3.8mmol) at room temperature. The reaction mixture was stirred 2h before being quenched with aqueous saturated ammonium chloride (5mL) and extracted three times with ethyl acetate (3x30mL). The combined organic layers were washed with brine (15mL), dried over solid Sodium sulfate, filtered and evaporated under reduced pressure yielding a brownish oil (436.8mg). QNMR indicated a strength of 18.4% w/w, leading to a chemical yield of 23%. ¹H NMR (400 MHz, d6-DMSO) δ ppm 1.80 - 1.84 (m, 2 H) 1.93 - 1.98 (m, 2 H) 8.13 (d, *J*=1.83 Hz, 1 H) 8.72 (d, *J*=1.83 Hz, 1 H) LC-MS: ret.: 0.81 min, m/z +H⁺: 204/206

### Example 4: Preparation of methyl 5-(1-cyanocyclopropyl)-3-ethylsulfanyl-pyridine-2-carboxylate

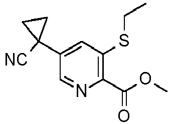

To a solution of methyl 5-bromo-3-ethylsulfanyl-pyridine-2-carboxylate (0.3g, 1.086mmol) and 5-amino-2,3-dihydrothiophene-4-carbonitrile (0.144g, 1.141mmol) in DMF (4.34mL) was added dropwise a solution of Sodium bis(trimethylsilyl)amide (1M in THF, 2.17mL, 2.17mmol) at room temperature. The reaction mixture was stirred 4h20 before being quenched with aqueous saturated ammonium chloride (10mL) and extracted three times with methyl tButyl ether (3x20mL). The combined organic layers were washed twice with water (2×10mL), then brine (10mL), dried over solid Sodium sulfate, filtered and evaporated under reduced pressure yielding a yellow oil (287mg). QNMR indicated a strength of 61% w/w, leading to a chemical yield of 61%. ¹H NMR (400 MHz, CDCl3) δ ppm 1.43 (t, *J*=7.34 Hz, 3 H) 1.55 - 1.60 (m, 2 H) 1.86 - 1.93 (m, 2 H) 3.00 (q, *J*=7.34 Hz, 2 H) 4.00 - 4.02 (m, 3 H) 7.72 (d, *J*=2.20 Hz, 1 H) 8.16 (d, *J*=2.20 Hz, 1 H) LC-MS: ret.: 0.85 min, m/z +H⁺: 263

### Example 5: Preparation of ethyl 5-(1-cyanocyclopropyl)pyridine-2-carboxylate

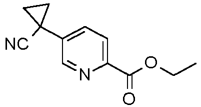

To a solution of ethyl 5-chloropyridine-2-carboxylate (0.098g, 0.527mmol) and 5-amino-2,3-dihydrothiophene-4-carbonitrile (0.066g, 0.527mmol) in DMF (2.11mL) was added dropwise a solution of Sodium bis(trimethylsilyl)amide (1M in THF, 1.05mL, 1.05mmol) at room temperature. The reaction mixture was stirred overnight at room temperature then 4h at 65°C before being allowed to cool down to room temperature. It was then quenched with aqueous saturated ammonium chloride (10mL), acidified with aqueous 1N HCl (10mL) and extracted five times with dichloromethane (5x30mL). The combined organic layers were dried over solid Sodium sulfate, filtered and evaporated under reduced pressure yielding an oil. QNMR indicated a chemical yield of 35%. The crude was purified by column chromatography to obtain the title compound as a white solid (38mg) in 34% isolated yield. ¹H NMR (400 MHz, CDCl₃): δ (ppm) = 8.62 (d, *J* = 1.8 Hz, 1H), 8.11 (d, *J* = 8.1 Hz, 1H), 7.76 (dd, *J* = 8.1, 2.6 Hz, 1H), 4.47 (q, *J* = 7.0 Hz, 2H), 1.97 - 1.80 (m, 2H), 1.62 - 1.47 (m, 2H), 1.43 (t, *J* = 7.2 Hz, 3H)

### Example 6: Preparation of ethyl 5-(1-cyanocyclopropyl)-3-ethylsulfanyl-pyridine-2-carboxylate

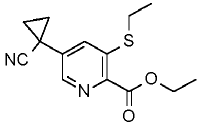

To a solution of ethyl 5-chloro-3-ethylsulfanyl-pyridine-2-carboxylate (0.1g, 0.372mmol) and 5-amino-2,3-dihydrothiophene-4-carbonitrile (0.049g, 0.391mmol) in DMF (1.5mL) was added dropwise a solution of Sodium bis(trimethylsilyl)amide (1M in THF, 0.74mL, 0.74mmol) at room temperature. The reaction mixture was stirred 3h30 before being quenched with aqueous saturated ammonium chloride (5mL) and extracted three times with methyl tButyl ether (3×10mL). The combined organic layers were washed twice with water (2×10mL), then brine (2×10mL), dried over solid Sodium sulfate, filtered and evaporated under reduced pressure yielding a yellow oil (113mg). QNMR indicated a strength of 66% w/w, leading to a chemical yield of 66%. ¹H NMR (400 MHz, CDCl3) δ ppm 1.40 - 1.47 (m, 6 H) 1.54-1.58 (m, 2 H) 1.88 - 1.93 (m, 2 H) 3.00 (q, *J*=7.34 Hz, 2 H) 4.48 (q, *J*=7.34 Hz, 2 H) 7.72 (d, *J*=2.20 Hz, 1 H) 8.18 (d, *J*=2.20 Hz, 1 H)

### Example 7: Preparation of ethyl 3-chloro-5-(1-cyanocyclopropyl)pyridine-2-carboxylate

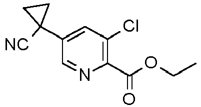

To a solution of ethyl 3,5-dichloropyridine-2-carboxylate (0.25g, 94.7% w/w purity, 1.08mmol) and 5-amino-2,3-dihydrothiophene-4-carbonitrile (0.143g, 1.13mmol) in DMF (4.3mL) was added dropwise a solution of Sodium bis(trimethylsilyl)amide (1M in THF, 2.15mL, 2.15mmol) at room temperature. The reaction mixture was stirred overnight before being quenched with aqueous saturated ammonium chloride (10mL) and extracted three times with methyl tButyl ether (3x20mL). The combined organic layers were washed twice with brine (2x20mL), dried over solid Sodium sulfate, filtered and evaporated under reduced pressure yielding a brownish oil (301mg). QNMR indicated a strength of 47% w/w, leading to a chemical yield of 52%. ¹H NMR (400 MHz, CDCl3) δ ppm 1.44 (t, *J*=7.15 Hz, 3 H) 1.53 - 1.57 (m, 2 H) 1.89 - 1.95 (m, 2 H) 4.48 (q, *J*=7.34 Hz, 2 H) 7.74 (d, *J*=2.20 Hz, 1 H) 8.47 (d, *J*=1.83 Hz, 1 H). LC-MS: ret.: 0.87 min, m/z +H⁺: 251/253

### Example 8: Preparation of isopropyl 3-chloro-5-(1-cyanocyclopropyl)pyridine-2-carboxylate

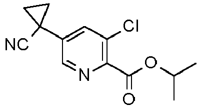

To a solution of isopropyl 3,5-dichloropyridine-2-carboxylate (0.1g, 98% w/w purity, 0.419mmol) and 5-amino-2,3-dihydrothiophene-4-carbonitrile (0.057g, 98% w/w purity, 0.44mmol) in DMF (1.7mL) was added dropwise a solution of Lithium bis(trimethylsilyl)amide (1M in THF, 0.84mL, 0.84mmol) at room temperature. The reaction mixture was stirred at this temperature for 2h45 then at 50°C for 30min, before being allowed to cool down to room temperature. It was then quenched with aqueous saturated ammonium chloride (5mL) and extracted three times with ethyl acetate (3x15mL). The combined organic layers were washed twice brine (2x20mL), dried over solid Sodium sulfate, filtered and evaporated under reduced pressure yielding a dark oil (440mg). QNMR indicated a strength of 12% w/w, leading to a chemical yield of 49%. ¹H NMR (400 MHz, CDCl3) δ ppm 1.39 (d, *J*=6.24 Hz, 6 H) 1.50 - 1.53 (m, 2 H) 1.85 - 1.89 (m, 2 H) 5.31 (hept, *J*=6.24 Hz, 1 H) 7.69 (d, *J*=2.20 Hz, 1 H) 8.43 (d, *J*=1.83 Hz, 1 H).

### Example 9: Preparation of tert-butyl 5-(1-cyanocyclopropyl)pyridine-2-carboxylate

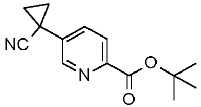

To a solution of tert-butyl 5-chloropyridine-2-carboxylate (0.169g, 0.792mmol) and 5-amino-2,3-dihydrothiophene-4-carbonitrile (0.1g, 0.792mmol) in DMF (3.1mL) was added dropwise a solution of Sodium bis(trimethylsilyl)amide (1M in THF, 1.58mL, 1.58mmol) at room temperature. The reaction mixture was stirred at this temperature for 6h. It was then quenched with aqueous saturated ammonium chloride (10mL), acidified with aqueous 1N HCl (10mL) and extracted four times with dichloromethane (4x30mL). The combined organic layers were dried over solid magnesium sulfate, filtered and evaporated under reduced pressure yielding a brown solid. QNMR indicated a chemical yield of 70%. The crude was purified by column chromatography to obtain the title compound as a pale solid (128mg) in 66% isolated yield. ¹H NMR (400 MHz, CDCl₃): δ (ppm) = 8.60 (d, *J* = 2.6 Hz, 1H), 8.02 (d, *J* = 7.7 Hz, 1H), 7.75 (dd, *J* = 8.3, 2.4 Hz, 1H), 1.90 - 1.85 (m, 2H), 1.62 (s, 9H), 1.55 - 1.49 (m, 2H).

### Example 10: Preparation of tert-butyl 3-chloro-5-(1-cyanocyclopropyl)pyridine-2-carboxylate

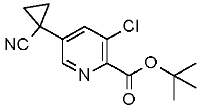

To a solution of tert-butyl 3,5-dichloropyridine-2-carboxylate (0.3g, 82% w/w purity, 0.993mmol) and 5-amino-2,3-dihydrothiophene-4-carbonitrile (0.134g, 94.3% w/w purity, 1mmol) in DMF (4mL) was added dropwise a solution of potassium tert-butanolate (1.6M in THF, 1.4mL, 2.18mmol) at 0°C. The reaction mixture was stirred at this temperature for 1h then at room temperature for 2h30min, then at 40°C for 30min before being allowed to cool down to room temperature. It was then quenched with aqueous saturated ammonium chloride (5mL) and extracted three times with ethyl acetate (3x20mL). The combined organic layers were washed with brine (10mL), dried over solid Sodium sulfate, filtered and evaporated under reduced pressure yielding a dark oil (0.417g). QNMR indicated a strength of 20% w/w, leading to a chemical yield of 30%. ¹H NMR (400 MHz, d6-DMSO) δ ppm 1.56 (s, 9 H) 1.70 - 1.80 (m, 1 H) 1.83 - 1.90 (m, 1 H) 7.97 (d, *J*=1.83 Hz, 1 H) 8.59 (d, *J*=1.83 Hz, 1 H).

### Example 11: Preparation of 5-(1-cyanocyclopropyl)-3-ethylsulfanyl-pyridine-2-carboxylic acid

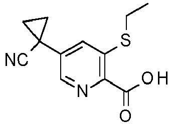

To a suspension of tBuONa (6.42g, 64.8mmol) in NMP (17.3mL) heated at 65°C was added a solution of 5-chloro-3-ethylsulfanyl-pyridine-2-carboxylic acid (5g, 94.1% w/w purity, 21.6mmol) and 5-amino-2,3-dihydrothiophene-4-carbonitrile (3.43g, 95.4% w/w purity, 25.9mmol) in NMP (21.6mL) over a period of 1h. After the end of addition, the mixture was stirred 20 minutes before an extra portion of NaOtBu (1.61g, 16.2mmol) was added. After one hour, still at 65°C, a short path distilling apparatus was fitted to the round bottom flask, (with Vigreux), the pressure was reduced to 20mbar and the mixture was stirred for an extra 1 h at 65°C (while distillation of the tBuOH occurred). After being allowed to come back at atmospheric pressure, a solution of MgCl₂ (3.23g, 54mmol) in NMP (17.3mL) was added in 5 minutes to the hot reaction mixture. After 1h, the mixture was allowed to cool down to room temperature; 50mL of 1N aq. NaOH and 50mL of water were added. The mixture was transferred to a separatory funnel and washed with TBME (100mL). The phases were separated, and the aqueous layer was then acidified to pH 1.5 with 1N HCl, and extracted three times with EtOAc (3x300mL). The combined organic layers (from EtOAc extractions) were dried over anhydrous sodium sulfate, filtered and evaporated under reduced pressure. 6mL of d6-DMSO were added to homogenize the concentrate to a clear, dark brown liquid (24.57g). Purity was measured by 1H-QNMR twice; to give a strength of 19.7% (therefore a chemical yield of 90%). The solution was then dropped onto ice cold water under stirring; a beige precipitate appeared and was collected (4.758g). Purity was measured to be 84% w/w; the isolated yield was therefore of 82%.

Alternatively, the same product can be obtained from 5-(3,3-dicyanopropylsulfanyl)-3-ethylsulfanyl-pyridine-2-carboxylic acid:
To a solution of 5-(3,3-dicyanopropylsulfanyl)-3-ethylsulfanyl-pyridine-2-carboxylic acid (5.2mmol) in 10mL of NMP was added a 50% aq. NaOH solution (0.728mL, 13.8mmol). The two phases were mixed vigorously for 30min then the flask was transferred to the rotavap, heated at 50°C under vacuum to remove the water. After this operation, extra NMP was added (5.2mL) and the mixture was heated at 65°C. MgClz (1g, 10.41mmol) was then added in one portion and the reaction was stirred at 65°C for 3h. An extra portion of MgClz was then added (0.25g, 2.6mmol) and the mixture was stirred for 30min at 65°C before being allowed to cool down to room temperature. The mixture was diluted with 1M NaOH, then acidified until pH 6-7 with 2N HCl before ethyl acetate was added. The pH of the aqueous was further lowered to 1.5 by addition of 2N HCl, the phases were separated and the aqueous phase was extracted three times with ethyl acetate. The combined organic layers were dried over anhydrous sodium sulfate, filtered and evaporated under reduced pressure. A dark oil (4.37g) was obtained. Purity was measured by 1H-QNMR; to give a strength of 26% (therefore a chemical yield of 88%). The solution was then dropped onto 75mL of ice cold water under stirring; a beige precipitate appeared and was collected (1.173g). Purity was measured to be 79% w/w; the isolated yield was therefore of 71%.
¹H NMR (400 MHz, d6-DMSO) δ ppm 1.25 (t, *J*=7.34 Hz, 3 H) 1.68 - 1.76 (m, 2 H) 1.81 - 1.89 (m, 2 H) 3.02 (q, *J*=7.34 Hz, 2 H) 7.62 (d, *J*=1.83 Hz, 1 H) 8.36 (d, *J*=2.20 Hz, 1 H)
LC-MS: m/z +H⁺: 249

### Example 12: Preparation of 3-chloro-5-(1-cyanocyclopropyl)pyridine-2-carboxylic acid

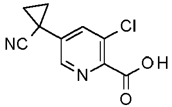

To a solution of 5-bromo-3-chloro-pyridine-2-carboxylic acid (0.3g, 1.269mmol) and 5-amino-2,3-dihydrothiophene-4-carbonitrile (0.178g, 94.3% w/w purity, 1.332mmol) in DMF (2.5mL) was added dropwise a solution of potassium tert-butanolate (1.7M in THF, 3mL, 5.08mmol) at room temperature. The reaction mixture was stirred at this temperature for 3h, then solid Magnesium dichloride (0.169g, 1.776mmol) was added. The reaction mixture was stirred 1h20min at 50°C before being allowed to cool down to room temperature. It was then quenched with 2N aqueous HCl to pH 1 and extracted three times with ethyl acetate (3x20mL). The combined organic layers were dried over solid Sodium sulfate, filtered and evaporated under reduced pressure yielding a dark oil (0.435g). QNMR indicated a strength of 38.4% w/w, leading to a chemical yield of 59%. ¹H NMR (400 MHz, d6-DMSO) δ ppm 1.71 - 1.76 (m, 2 H) 1.83 - 1.88 (m, 2 H) 7.93 (d, *J*=2.20 Hz, 1 H) 8.58 (d, *J*=1.83 Hz, 1 H) LC-MS: m/z +H⁺: 223/225

### Example 13: Preparation of 5-(1-cyanocyclopropyl)-3-ethylsulfanyl-pyridine-2-carboxamide

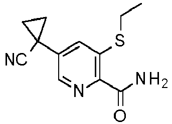

A solution of 5-chloro-3-ethylsulfanyl-pyridine-2-carboxamide (0.2g, 98% purity w/w, 0.905mmol) and 5-amino-2,3-dihydrothiophene-4-carbonitrile (0.156g, 95% w/w purity, 1.18mmol) in DMF (3.6mL) at 65°C was added to a solution of Sodium tert-butanolate (0.179g, 1.81mmol) in 3.6mL of DMF at 65°C. The reaction mixture was stirred at this temperature overnight. The compound can be characterized after standard work-up procedure. ¹H NMR (400 MHz, d6-DMSO) δ ppm 1.26 (t, *J*=7.34 Hz, 3 H) 1.70 - 1.74 (m, 2 H) 1.84 - 1.87 (m, 2 H) 2.95 (q, *J*=7.34 Hz, 2 H) 7.57 (d, *J*=1.83 Hz, 1 H) 7.57 (bs, 1H) 7.99 (bs, 1 H) 8.28 (d, *J*=2.20 Hz, 1 H)

### Example 14: Preparation of 2-[2-[(6-cyano-5-ethylsulfanyl-3-pyridyl)sulfanyl]ethyl]propanedinitrile

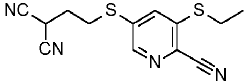

To a solution of 5-bromo-3-ethylsulfanyl-pyridine-2-carbonitrile (0.2g, 0.823mmol) and 5-amino-2,3-dihydrothiophene-4-carbonitrile (0.135g, 92.4% w/w purity, 0.987mmol) in DMF (3.2mL) cooled at-20°C was added dropwise a solution of potassium tert-butanolate (1.6M in THF, 1.1mL, 1.727mmol). The reaction mixture was stirred at this temperature for 1h, then quenched with aqueous saturated ammonium chloride (5mL) and extracted three times with ethyl acetate (3x20mL). The combined organic layers were washed twice with brine (2×10mL), dried over solid Sodium sulfate, filtered and evaporated under reduced pressure yielding a dark oil (0.288g). QNMR indicated a strength of 47% w/w, leading to a chemical yield of 57%. ¹H NMR (400 MHz, d6-DMSO) δ ppm 1.28 (t, *J*=7.34 Hz, 3 H) 2.36 - 2.42 (m, 2 H) 3.22 (q, *J*=7.34 Hz, 2 H) 3.30 - 3.37 (m, 2 H) 4.91 (t, *J*=6.79 Hz, 1 H), 7.86 (d, *J*=1.83 Hz, 1 H) 8.45 (d, *J*=1.83 Hz, 1 H) LC-MS: m/z +H⁺: 289

### Example 15: Preparation of methyl 5-(3,3-dicyanopropylsulfanyl)-3-ethylsulfanyl-pyridine-2-carboxylate

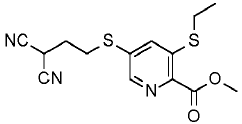

To a solution of methyl 5-bromo-3-ethylsulfanyl-pyridine-2-carboxylate (0.3g, 1.086mmol) and 5-amino-2,3-dihydrothiophene-4-carbonitrile (0.141g, 1.12mmol) in DMF (2.2mL) cooled at 0°C was added dropwise a solution of Sodium bis(trimethylsilyl)amide (1M in THF, 2.4mL, 2.4mmol). The reaction mixture was stirred at this temperature for 45min, then quenched with aqueous saturated ammonium chloride (5mL) and extracted three times with ethyl acetate (3x20mL). The combined organic layers were washed twice with brine (2×10mL), dried over solid Sodium sulfate, filtered and evaporated under reduced pressure yielding a dark oil (0.346g). QNMR indicated a strength of 75% w/w, leading to a chemical yield of 74%. ¹H NMR (400 MHz, d6-DMSO) δ ppm 1.25 (t, *J*=7.34 Hz, 3 H) 2.35 - 2.41 (m, 2 H) 3.05 (q, *J*=7.34 Hz, 2 H) 3.29 - 3.34 (m, 2 H) 3.85 (s, 3 H) 4.93 (t, *J*=6.97 Hz, 1 H) 7.73 (d, *J*=2.20 Hz, 1 H) 8.35 (d, *J*=1.83 Hz, 1 H) LC-MS: m/z +H⁺: 322

### Example 16: Preparation of ethyl 5-(3,3-dicyanopropylsulfanyl)-3-ethylsulfanyl-pyridine-2-carboxylate

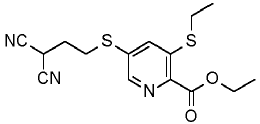

To a solution of ethyl 5-chloro-3-ethylsulfanyl-pyridine-2-carboxylate (0.2g, 0.814mmol) and 5-amino-2,3-dihydrothiophene-4-carbonitrile (0.112g, 96.3% w/w purity, 0.855mmol) in DMF (3.2mL) cooled at - 20°C was added dropwise a solution of Sodium bis(trimethylsilyl)amide (1M in THF, 1.8mL, 1.8mmol). The reaction mixture was stirred at this temperature for 1h, then quenched with aqueous saturated ammonium chloride (5mL) and extracted three times with ethyl acetate (3x20mL). The combined organic layers were washed twice with brine (2×10mL), dried over solid Sodium sulfate, filtered and evaporated under reduced pressure yielding a dark oil (0.316g). QNMR indicated a strength of 47% w/w, leading to a chemical yield of 54%. ¹H NMR (400 MHz, CDCl3) δ ppm 1.40 (t, *J*=7.34 Hz, 3 H) 1.43 - 1.47 (t, 3 H) 2.37 (q, *J*=7.09 Hz, 2 H) 2.92 - 2.95 (m, 2 H) 3.25 - 3.30 (m, 2 H) 4.14 - 4.18 (t, 1 H) 4.45 - 4.50 (m, 2 H) 7.59 (d, *J*=2.20 Hz, 1 H) 8.35 (d, *J*=2.20 Hz, 1 H) LC-MS: m/z +H⁺: 336

### Example 17: Preparation of ethyl 3-chloro-5-(3,3-dicyanopropylsulfanyl)pyridine-2-carboxylate

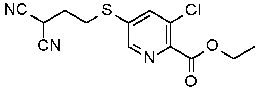

To a solution of ethyl 3,5-dichloropyridine-2-carboxylate (0.2g, 0.861mmol) and 5-amino-2,3-dihydrothiophene-4-carbonitrile (0.118g, 96.3% w/w purity, 0.904mmol) in DMF (3.4mL) cooled at-20°C was added dropwise a solution of Sodium bis(trimethylsilyl)amide (1M in THF, 1.9mL, 1.9mmol). The reaction mixture was stirred at this temperature for 1h, then quenched with aqueous saturated ammonium chloride (5mL) and extracted three times with TBME (3x20mL). The combined organic layers were washed twice with brine (2×10mL), dried over solid Sodium sulfate, filtered and evaporated under reduced pressure yielding a dark oil (0.296g). QNMR indicated a strength of 37% w/w, leading to a chemical yield of 41%. ¹H NMR (400 MHz, CDCl3) δ ppm 1.44 (t, *J*=7.15 Hz, 3 H) 2.36 - 2.42 (m, 2 H) 3.28 (t, *J*=7.15 Hz, 2 H) 4.17 (t, *J*=7.15 Hz, 1 H) 4.45 - 4.51 (q, 2 H) 7.76 (d, *J*=2.20 Hz, 1 H) 8.49 (d, *J*=1.83 Hz, 1 H) LC-MS: ret.: 0.93 min, m/z +H⁺: 310/312

### Example 18: Preparation of 5-(3,3-dicyanopropylsulfanyl)-3-ethylsulfanyl-pyridine-2-carboxylic acid

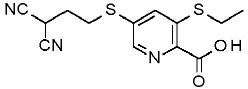

To a solution of 5-chloro-3-ethylsulfanyl-pyridine-2-carboxylic acid (1.5g, 92% w/w purity, 6.65mmol) and 5-amino-2,3-dihydrothiophene-4-carbonitrile (1.1g, 94.3% w/w purity, 8.25mmol) in DMF (25mL) was added a solution of Lithium bis(trimethylsilyl)amide (1M in THF, 19mL, 19mmol) over a period of 30min at room temperature. The reaction mixture was stirred at this temperature for 1h. It was then quenched with aqueous 1N aqueous HCl (22ml) extracted three times with ethyl acetate (3x100mL). The combined organic layers were washed twice brine (2x50mL), dried over solid Sodium sulfate, filtered and evaporated under reduced pressure yielding an oil which solidified over time (2.582g). QNMR indicated a strength of 70% w/w, leading to a chemical yield of 92%.

### Alternatively, the title product can be obtained using the following protocol:

Solid sodium tert-butoxide (19.3g, 97% w/w, 195mmol) was suspended in NMP (65mL), the mixture was then stirred at 67°C until a turbid solution was formed. To this solution, a mixture of 5-chloro-3-ethylsulfanyl-pyridine-2-carboxylic acid (15g, 94.1%w/w purity, 64.8mmol) and 5-amino-2,3-dihydrothiophene-4-carbonitrile (10.3g, 95.4% w/w purity, 77.8mmol) in NMP (65mL) was added dropwise over a period of 1h. After addition, solid sodium tert-butoxide (3.21g, 97% w/w, 32.4mmol) was added and the mixture stirred for 1.5h, before being cooled down to room temperature. 150mL of 1M aq. NaOH was added and the mixture was stirred for 10 minutes before being diluted with 100mL of TBME (tert-butyl-methyl-ether). The phases were separated and the aqueous phase, containing the desired product, was acidified with 4N aq. HCl until a pH of 1.5 to 1 was reached. The aqueous phase was extracted three times with EtOAc (3x150mL). The combined organic layers (from EtOAc) were dried over solid anhydrous sodium sulfate and concentrated under reduced pressure to give a crude solution (57.57g) of desired product 5-(3,3-dicyanopropylsulfanyl)-3-ethylsulfanyl-pyridine-2-carboxylic acid in residual NMP. Quantification led to a measurement of 32%w/w purity in NMP, leading to a chemical yield of 92.4%.

1H NMR (400 MHz, d6-DMSO) δ ppm 1.25 (t, J=7.34 Hz, 3 H) 2.36 (q, J=7.09 Hz, 2 H) 3.02 (q, J=7.34 Hz, 2 H) 3.30 (dd, J=8.44, 6.60 Hz, 2 H) 4.92 (t, J=6.97 Hz, 1 H) 7.70 (d, J=2.20 Hz, 1 H) 8.33 (d, J=2.20 Hz, 1 H) 13.12 (br s, 1 H) LC-MS: m/z +H+: 308

### Example 19: Preparation of 3-chloro-5-(3,3-dicyanopropylsulfanyl)pyridine-2-carboxylic acid

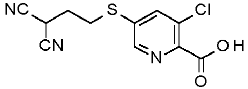

To a solution of 5-bromo-3-chloro-pyridine-2-carboxylic acid (0.3g, 1.27mmol) and 5-amino-2,3-dihydrothiophene-4-carbonitrile (0.165g, 1.307mmol) in DMF (2.5mL) cooled at -20°C was added dropwise a solution of Sodium bis(trimethylsilyl)amide (1M in THF, 1.9mL, 1.9mmol). The reaction mixture was stirred at room temperature for 3h, then quenched with aqueous saturated ammonium chloride (5mL), pH was adjusted to 1 by addition of aq. 2N HCl, and this was extracted three times with ethyl acetate (3x20mL). The combined organic layers were washed twice with brine (2x10mL), dried over solid Sodium sulfate, filtered and evaporated under reduced pressure yielding a dark oil (0.456g). QNMR indicated a strength of 35% w/w, leading to a chemical yield of 44%. ¹H NMR (400 MHz, d6-DMSO) δ ppm 2.32 - 2.41 (m, 2 H) 3.29 (dd, *J*=8.25, 6.79 Hz, 2 H) 4.91 (t, *J*=6.97 Hz, 1 H) 8.10 (d, *J*=1.83 Hz, 1 H) 8.53 (d, *J*=1.83 Hz, 1 H) 13.44 - 13.99 (m, 1 H) 13.69 (s, 1 H) LC-MS: m/z +H⁺: 282/284

### Example 20: Preparation of 5-(3,3-dicyanopropylsulfanyl)-3-ethylsulfanyl-pyridine-2-carboxamide

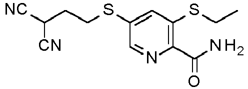

To a solution of 5-chloro-3-ethylsulfanyl-pyridine-2-carboxamide (0.2g, 98% purity w/w, 0.905mmol) and 5-amino-2,3-dihydrothiophene-4-carbonitrile (0.156g, 95% purity w/w, 1.18mmol) in DMF (3.6mL) was added Sodium tert-butoxide (0.358g, 3.62mmol). The reaction mixture was stirred at room temperature for 2h, then quenched by pouring onto ice/water, and this was extracted three times with ethyl acetate. The combined organic layers were dried over solid Sodium sulfate, filtered and evaporated under reduced pressure yielding a pale yellow solid (0.319g). Ethyl acetate was added to the solid, this solid was then triturated and ethyl acetate was removed by filtration. The white solid was collected (94mg), leading to a chemical yield of 34%. ¹H NMR (400 MHz, d6-DMSO) δ ppm 1.26 (t, *J*=7.34 Hz, 3 H) 2.36 (q, *J*=6.97 Hz, 2 H) 2.95 (q, *J*=7.34 Hz, 2 H) 3.28 (m, 2 H) 4.93 (t, *J*=6.97 Hz, 1 H) 7.51 (bs, 1H) 7.66 (d, *J*=1.47 Hz, 1 H) 7.95 (bs, 1H) 8.28 (d, *J*=1.47 Hz, 1 H)

### Example 21: Preparation of 5-(3-cyano-2-imino-tetrahydrothiophen-3-yl)-3-ethylsulfanyl-pyridine-2-carboxylic acid

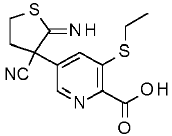

To a solution of 5-(1-cyanocyclopropyl)-3-ethylsulfanyl-pyridine-2-carboxylic acid (1g, 4.027mmol), acetic acid (0.048g, 0.805mmol), tetrabutylammonium bromide (0.26g, 0.805mmol) in DMF (8mL) was added potassium thiocyanate (1.96g, 20.14mmol). The mixture was stirred overnight at 85°C, then 3h at 105°C before being cooled down to room temperature. The reaction mixture was then poured into an acetonitrile:water mixture containing 0.1% of acetic acid. The solvent was evaporated yielding a brownish wax (6.782g). To 1g of this wax was added aqueous acetonitrile and ethyl acetate, the upper layer was decanted off and washed three times with ethyl acetate. The oil was then dried under reduced pressure. ¹H NMR (400 MHz, d6-DMSO) δ ppm 1.19 (t, *J*=7.34 Hz, 3 H) 2.89 (m, 3H) 3.01 (dt, J=11 , 5.14 Hz, 1H) 3.17 (m, 1H) 3.40 (dt, J=11.37, 5.87 Hz, 1H) 7.69 (d, J=2.2 Hz, 1H) 8.38 (d, J=1.8 Hz, 1H) 11.31 (s, 1H)

### Example 22: Preparation of 5-(1-cyano-3-thiocyanato-propyl)-3-ethylsulfanyl-pyridine-2-carboxylic acid

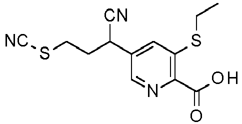

To a solution of 5-(1-cyanocyclopropyl)-3-ethylsulfanyl-pyridine-2-carboxylic acid (1.5g, 73.1 % w/w purity, 4.4mmol), dissolved in dioxane (6.6mL), acetic acid (6.6mL) was added Hydrochloric acid (1M in dioxane, 4.4mL, 4.4mmol). The reaction was stirred overnight at 75°C, before being allowed to cool down to room temperature. 10mL of 2N aq. HCl were added, 20mL of EtOAc were added, and the phases separated. The aqueous phase was extracted twice with 20mL of EtOAc, combined, dried over solid Sodium sulfate, filtered and evaporated under reduced pressure yielding a brown oil (1.5525g).

The crude 5-(3-chloro-1-cyano-propyl)-3-ethylsulfanyl-pyridine-2-carboxylic acid obtained (purity measured 63.3% ; chemical yield 78%) was then purified by column chromatography ; the purity was then increased to 86.7% w/w. 0.65g of this product 5-(3-chloro-1-cyano-propyl)-3-ethylsulfanyl-pyridine-2-carboxylic acid (86.7% w/w pure, 1.98mmol) were then dissolved in DMF (5.94mL) ; potassium thiocyanate (0.5g, 5.14mmol) was added and the reaction mixture was heated at 70°C overnight. Extra potassium thiocyanate (0.25g, 2.57mmol) were added and the reaction mixture was stirred 4h at 70°C before being allowed to cool down to room temperature. The mixture was acidified to pH 1 with 2N aq. HCl and extracted three times with EtOAc (3x30mL). The combined organic layers were then dried over anhydrous sodium sulfate, filtered and evaporated under reduced pressure yielding a brown oil (1.2157g). QNMR indicated a purity of 74.2% w/w, the desired title compound was then obtained with a chemical yield of 74%. ¹H NMR (400 MHz, d6-DMSO) δ ppm 1.25 - 1.30 (m, 3 H) 2.33 - 2.44 (m, 1 H) 2.52 - 2.58 (m, 1 H) 3.03 (q, *J*=7.34 Hz, 2 H) 3.14 - 3.20 (m, 2 H) 4.60 (dd, *J*=8.07, 6.60 Hz, 1 H) 7.93 (d, *J*=1.83 Hz, 1 H) 8.46 (d, *J*=1.83 Hz, 1 H) LC-MS: m/z +H⁺: 308

### Example 23: Preparation of 5-(1-cyanocyclopropyl)-N-methyl-pyridine-2-carboxamide

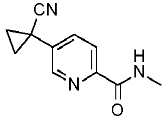

To a solution of 5-chloro-N-methyl-pyridine-2-carboxamide (0.1g, 0.584mmol), 5-amino-2,3-dihydrothiophene-4-carbonitrile (0.78g, 94%w/w purity, 0.584mmol) in DMF (4.67mL) is added a 2M THF solution of sodium tertiobutanolate (0.64mL, 1.28mmol). The reaction mixture is heated at 70°C overnight, then cooled down to room temperature. 10mL of an aqueous saturated NH4Cl solution is added into the mixture, which is then acidified by 10mL of 1N aq. HCl. The mixture is extracted three times with EtOAc (3x20mL), the combined organic layers are washed with 10mL of sat. aq. NaHCO3, then twice with 10mL of brine. The combined organic layers are then dried over anhydrous magnesium sulfate and concentrated under reduced pressure, giving 0.1g of an orange oil. The crude is then purified by column chromatography, affording 30.1mg of 5-(1-cyanocyclopropyl)-N-methylpyridine-2-carboxamide under the form of a white solid (isolated yield of 25%). 1H NMR (400 MHz, CDCl₃) δ ppm 1.45 - 1.57 (m, 2 H) 1.84 - 1.92 (m, 2 H) 3.06 (d, J=5.09 Hz, 3 H) 7.70 (dd, J=8.17, 2.36 Hz, 1 H) 7.96 (br s, 1 H) 8.20 (d, J=7.99 Hz, 1 H) 8.57 (d, J=2.18 Hz, 1 H)

## Claims

1. A process for the preparation of a compound of formula I: 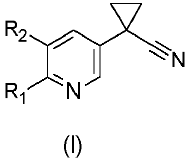 wherein R₁ is -CO₂R₄, -CO(NR₅R₆), carboxylate or cyano; R₂ is hydrogen, halogen or -SR₃; R₃ is C₁-C₄alkyl or C₃-C₆cycloalkyl-C₁-C₄alkyl; R₄ is hydrogen, -Si(CH₃)₃ or C₁-C₆alkyl; and R₅ and R₆ are, independently from each other, hydrogen or C₁-C₄alkyl, or an agrochemically acceptable salt of a compound of formula I; which process comprises:
reacting a compound of formula (III) 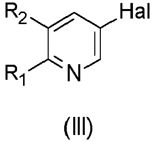
wherein R₁ and R₂ are defined as under formula I above and Hal is halogen
with a compound of formula (IV), 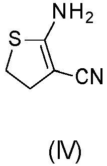
in the presence of a suitable base, in an appropriate solvent (or diluent);
to produce a compound of formula (I) or an agrochemically acceptable salt thereof.

2. The process according to claim 1, wherein R₁ is -CO₂R₄, -CONH₂, carboxylate or a cyano group; and R₄ is hydrogen, -Si(CH₃)₃, methyl, ethyl, isopropyl, tert-butyl, or sec-butyl.

3. The process according to claim 2, wherein R₁ is -CO₂H or carboxylate.

4. The process according to any one of the previous claims, wherein R₂ is hydrogen, chloro or -SR₃; wherein R₃ is ethyl.

5. The process according to claim 4, wherein R₂ is -S-ethyl.

6. The process according to any one of the previous claims, wherein Hal is chloro or bromo.

7. The process according to any one of the previous claims, wherein the suitable base is selected from sodium hydroxide, potassium hydroxide, sodium tertiobutanolate, and potassium tertiobutanolate.

8. The process according to any one of the previous claims, wherein the solvent (or diluent) is selected from dimethylformamide, dimethylsulfoxide, N-methyl-pyrrolidine, dimethylacetamide, sulfolane and N,N'-dimethylpropyleneurea (DMPU).

9. The process according to any one of the previous claims, which is carried out in a temperature range from approximately 0°C to approximately +100°C, preferably from approximately +20°C to approximately +80°C.

10. The process according to any one of claims 1 - 6, 8 or 9, wherein, when R₁ is a carboxylate anion, the suitable base is selected from magnesium bis(tertio-butanolate) and magnesium bis(hexamethyldisilazide).

11. The process according to any one of claims 1 - 6, 8 or 9, wherein, when R₁ is a carboxylate anion, the suitable base is selected from sodium hydroxide, potassium hydroxide, sodium tertiobutanolate, and potassium tertiobutanolate, and wherein the process further comprises addition of magnesium^{(II)}, zinc ^{(II)} or Al^{(III)} salts as additives following *in situ* formation of a compound of formula (INT I) 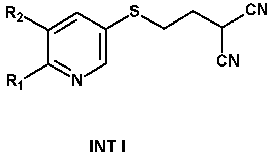 wherein R₁ and R₂ are as defined in formula I in claim 1.

12. The process according to claim 11, wherein the magnesium^{(II)}, zinc ^{(II)} or Al^{(III)} salts are selected from MgCl₂, ZnCl₂, Al(OtBu)₃ and AlCl₃.

13. The process according to any one of claims 1 - 6, 8 or 9, wherein, when R₁ is a carboxylate anion, the suitable base is selected from sodium hydroxide, potassium hydroxide, sodium tertiobutanolate, and potassium tertiobutanolate, and wherein the process further comprises addition of trimethylsilylchloride after distillation of the water or alcohol generated during the *in situ* formation of a compound of formula (INT I) 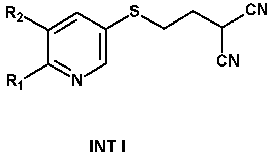 wherein R₁ and R₂ are as defined in formula I in claim 1.

14. A compound of formula (INT I) 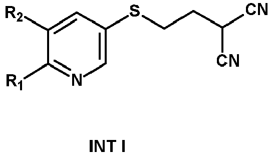 wherein R₁ is -CO₂R₄, -CO(NR₅R₆) or cyano; R₂ is hydrogen, halogen or -SR₃; R₃ is C₁-C₄alkyl or C₃-C₆cycloalkyl-C₁-C₄alkyl; R₄ is hydrogen, -Si(CH₃)₃ or C₁-C₆alkyl; and R₅ and R₆ are, independently from each other, hydrogen or C₁-C₄alkyl; or an agrochemically acceptable salt of a compound of formula INT I.

15. A compound of formula (INT II) 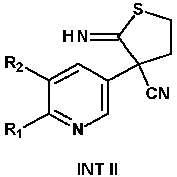 wherein R₁ is -CO₂R₄, -CO(NR₅R₆) or cyano; R₂ is hydrogen, halogen or -SR₃; R₃ is C₁-C₄alkyl or C₃-C₆cycloalkyl-C₁-C₄alkyl; R₄ is hydrogen, -Si(CH₃)₃ or C₁-C₆alkyl; and R₅ and R₆ are, independently from each other, hydrogen or C₁-C₄alkyl; or an agrochemically acceptable salt of a compound of formula INT II.

16. A compound of formula (INT III) 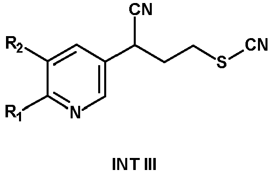 wherein R₁ is -CO₂R₄, -CO(NR₅R₆) or cyano; R₂ is hydrogen, halogen or -SR₃; R₃ is C₁-C₄alkyl or C₃-C₆cycloalkyl-C₁-C₄alkyl; R₄ is hydrogen, -Si(CH₃)₃ or C₁-C₆alkyl; and R₅ and R₆ are, independently from each other, hydrogen or C₁-C₄alkyl; or an agrochemically acceptable salt of a compound of formula INT III.

17. A compound according to any one of claims 14, 15 or 16, wherein R₁ is -CO₂R₄, -CONH₂ or cyano; wherein R₄ is hydrogen, -Si(CH₃)₃, methyl, ethyl, isopropyl, tert-butyl, or sec-butyl; and R₂ is hydrogen, chloro or -SR₃; wherein R₃ is ethyl.

18. A compound of formula INT I according to claim 14, selected from the group consisting of: 2-[2-[(6-cyano-5-ethylsulfanyl-3-pyridyl)sulfanyl]ethyl]propanedinitrile (example 14); methyl 5-(3,3-dicyanopropylsulfanyl)-3-ethylsulfanyl-pyridine-2-carboxylate (example 15); ethyl 5-(3,3-dicyanopropylsulfanyl)-3-ethylsulfanyl-pyridine-2-carboxylate (example 16); ethyl 3-chloro-5-(3,3-dicyanopropylsulfanyl)pyridine-2-carboxylate (example 17); 5-(3,3-dicyanopropylsulfanyl)-3-ethylsulfanyl-pyridine-2-carboxylic acid (example 18); 3-chloro-5-(3,3-dicyanopropylsulfanyl)pyridine-2-carboxylic acid (example 19); and 5-(3,3-dicyanopropylsulfanyl)-3-ethylsulfanyl-pyridine-2-carboxamide (example 20); a compound of formula INT II according to claim 15, selected from 5-(3-cyano-2-imino-tetrahydrothiophen-3-yl)-3-ethylsulfanyl-pyridine-2-carboxylic acid (example 21); or a compound of formula INT III according to claim 16, selected from 5-(1-cyano-3-thiocyanato-propyl)-3-ethylsulfanyl-pyridine-2-carboxylic acid (example 22).

19. A compound of formula Ia 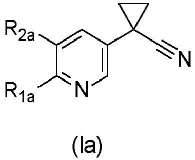
wherein R₁ₐ is -CO₂R₄ₐ, -CO(NR₅ₐR₆ₐ), carboxylate or cyano; R₂ₐ is hydrogen, halogen or -SR₃ₐ; R₃ₐ is C₁-C₄alkyl or C₃-C₆cycloalkyl-C₁-C₄alkyl; R₄ₐ is hydrogen, -Si(CH₃)₃ or C₁-C₆alkyl; and R₅ₐ and R₆ₐ are, independently from each other, hydrogen or C₁-C₄alkyl, or an agrochemically acceptable salt of a compound of formula la;
with the proviso that the compound of formula Ia is not: selected from the group consisting of a compound of the formula Ib, Ic and Id; 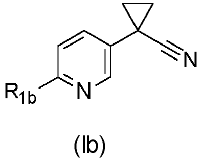
wherein R_{1b} is CN or-CO₂Me; 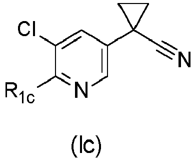
wherein R_{1c} is -CO₂H, -CO₂Me, -CO₂Et or -CONHMe; and 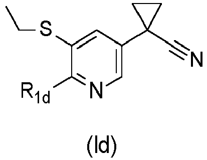
wherein R_{1d} is -CO₂H, -CO₂Me or -CONHMe.

20. A process for the preparation of a compound of formula I: 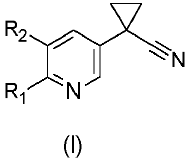 wherein R₁ is -CO₂R₄, -CO(NR₅R₆), carboxylate or cyano; R₂ is hydrogen, halogen or -SR₃; R₃ is C₁-C₄alkyl or C₃-C₆cycloalkyl-C₁-C₄alkyl; R₄ is hydrogen, -Si(CH₃)₃ or C₁-C₆alkyl; and R₅ and R₆ are, independently from each other, hydrogen or C₁-C₄alkyl, or an agrochemically acceptable salt of a compound of formula I; which process comprises:
reacting a compound of formula (INT I) 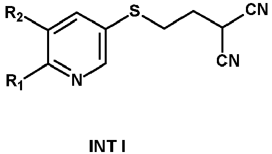
wherein R₁ and R₂ are defined as under formula I above in the presence of a suitable base, in an appropriate solvent (or diluent); to produce a compound of formula (I) or an agrochemically acceptable salt thereof.

## Patentansprüche

1. Verfahren zur Herstellung einer Verbindung der Formel I: 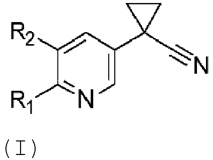 wobei R₁ für -CO₂R₄, -CO(NR₅R₆), Carboxylat oder Cyano steht; R₂ für Wasserstoff, Halogen oder -SR₃ steht; R₃ für C₁-C₄-Alkyl oder C₃-C₆-Cycloalkyl-C₁-C₄-alkyl steht; R₄ für Wasserstoff, -Si(CH₃)₃ oder C₁-C₆-Alkyl steht und R₅ und R₆ unabhängig voneinander für Wasserstoff oder C₁-C₄-Alkyl stehen, oder eines agrochemisch unbedenklichen Salzes einer Verbindung der Formel I; wobei das Verfahren Folgendes umfasst:
Umsetzen einer Verbindung der Formel (III), 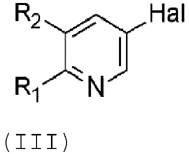
wobei R₁ und R₂ wie unter Formel I oben definiert sind und Hal für Halogen steht,
mit einer Verbindung der Formel (IV) 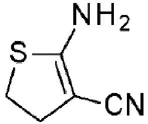 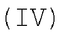
in Gegenwart einer geeigneten Base in einem passenden Lösungsmittel (oder Verdünnungsmittel);
zur Herstellung einer Verbindung der Formel (I) oder eines agrochemisch unbedenklichen Salzes davon.

2. Verfahren nach Anspruch 1, wobei R₁ für -CO₂R₄,-CONH₂, Carboxylat oder eine Cyanogruppe steht und R₄ für Wasserstoff, -Si(CH₃)₃, Methyl, Ethyl, Isopropyl, tert-Butyl oder sec-Butyl steht.

3. Verfahren nach Anspruch 2, wobei R₁ für -CO₂H oder Carboxylat steht.

4. Verfahren nach einem der vorhergehenden Ansprüche, wobei R₂ für Wasserstoff, Chlor oder -SR₃ steht;
wobei R₃ für Ethyl steht.

5. Verfahren nach Anspruch 4, wobei R₂ für -S-Ethyl steht.

6. Verfahren nach einem der vorhergehenden Ansprüche, wobei Hal für Chlor oder Brom steht.

7. Verfahren nach einem der vorhergehenden Ansprüche, wobei die geeignete Base aus Natriumhydroxid, Kaliumhydroxid, Natrium-tert-butanolat und Kalium-tert-butanolat ausgewählt wird.

8. Verfahren nach einem der vorhergehenden Ansprüche, wobei das Lösungsmittel (oder Verdünnungsmittel) aus Dimethylformamid, Dimethylsulfoxid, N-Methylpyrrolidin, Dimethylacetamid, Sulfolan und N,N'-Dimethylpropylenharnstoff (DMPU) ausgewählt wird.

9. Verfahren nach einem der vorhergehenden Ansprüche, das in einem Temperaturbereich von ungefähr 0 °C bis ungefähr +100 °C, vorzugsweise von ungefähr +20 °C bis ungefähr +80 °C, durchgeführt wird.

10. Verfahren nach einem der Ansprüche 1 - 6, 8 oder 9, wobei dann, wenn R₁ für ein Carboxylat-Anion steht, die geeignete Base aus Magnesiumbis(tert-butanolat) und Magnesiumbis(hexamethyldisilazid) ausgewählt wird.

11. Verfahren nach einem der Ansprüche 1 - 6, 8 oder 9, wobei dann, wenn R₁ für ein Carboxylat-Anion steht, die geeignete Base aus Natriumhydroxid, Kaliumhydroxid, Natrium-tert-butanolat und Kalium-tert-butanolat ausgewählt wird und wobei das Verfahren ferner das Zugeben von Magnesium^{(II)}-, Zink^{(II)}- oder Al^{(III)}-Salzen als Additive nach der Bildung einer Verbindung der Formel (INT I) in situ umfasst: 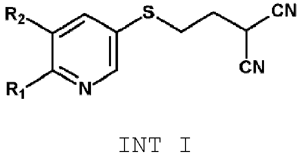 wobei R₁ und R₂ wie in Formel I in Anspruch 1 definiert sind.

12. Verfahren nach Anspruch 11, wobei die Magnesium^{(II)}-, Zink^{(II)}- oder Al^{(III)}-Salze aus MgCl₂, ZnCl₂, Al(OtBu)₃ und AlCl₃ ausgewählt werden.

13. Verfahren nach einem der Ansprüche 1 - 6, 8 oder 9, wobei dann, wenn R₁ für ein Carboxylat-Anion steht, die geeignete Base aus Natriumhydroxid, Kaliumhydroxid, Natrium-tert-butanolat und Kalium-tert-butanolat ausgewählt wird und wobei das Verfahren ferner das Zugeben von Trimethylsilylchlorid nach Abdestillieren des bei der Bildung der Verbindung der Formel (INT I) in situ erzeugten Wassers bzw. Alkohols umfasst: 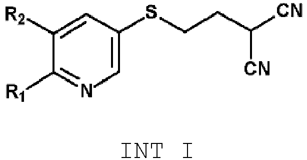 wobei R₁ und R₂ wie in Formel I in Anspruch 1 definiert sind.

14. Verbindung der Formel (INT I), 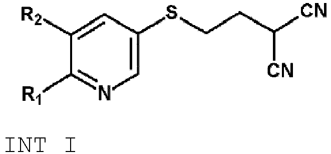 wobei R₁ für -CO₂R₄, -CO(NR₅R₆) oder Cyano steht; R₂ für Wasserstoff, Halogen oder -SR₃ steht; R₃ für C₁-C₄-Alkyl oder C₃-C₆-Cycloalkyl-C₁-C₄-alkyl steht; R₄ für Wasserstoff, -Si(CH₃)₃ oder C₁-C₆-Alkyl steht und R₅ und R₆ unabhängig voneinander für Wasserstoff oder C₁-C₄-Alkyl stehen, oder ein agrochemisch unbedenkliches Salz einer Verbindung der Formel INT I.

15. Verbindung der Formel (INT II), 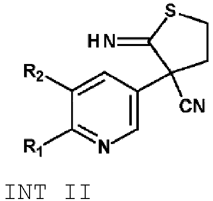 wobei R₁ für -CO₂R₄, -CO(NR₅R₆) oder Cyano steht; R₂ für Wasserstoff, Halogen oder -SR₃ steht; R₃ für C₁-C₄-Alkyl oder C₃-C₆-Cycloalkyl-C₁-C₄-alkyl steht; R₄ für Wasserstoff, -Si(CH₃)₃ oder C₁-C₆-Alkyl steht und R₅ und R₆ unabhängig voneinander für Wasserstoff oder C₁-C₄-Alkyl stehen, oder ein agrochemisch unbedenkliches Salz einer Verbindung der Formel INT II.

16. Verbindung der Formel (INT III), 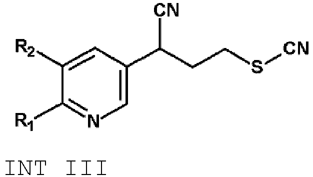 wobei R₁ für -CO₂R₄, -CO(NR₅R₆) oder Cyano steht; R₂ für Wasserstoff, Halogen oder -SR₃ steht; R₃ für C₁-C₄-Alkyl oder C₃-C₆-Cycloalkyl-C₁-C₄-alkyl steht; R₄ für Wasserstoff, -Si(CH₃)₃ oder C₁-C₆-Alkyl steht und R₅ und R₆ unabhängig voneinander für Wasserstoff oder C₁-C₄-Alkyl stehen, oder ein agrochemisch unbedenkliches Salz einer Verbindung der Formel INT III.

17. Verbindung nach einem der Ansprüche 14, 15 oder 16, wobei R₁ für -CO₂R₄, -CONH₂ oder Cyano steht; wobei R₄ für Wasserstoff, -Si(CH₃)₃, Methyl, Ethyl, Isopropyl, tert-Butyl oder sec-Butyl steht und R₂ für Wasserstoff, Chlor oder -SR₃ steht; wobei R₃ für Ethyl steht.

18. Verbindung der Formel INT I nach Anspruch 14, ausgewählt aus der Gruppe bestehend aus: 2-[2-[(6-Cyano-5-ethylsulfanyl-3-pyridyl)sulfanyl]ethyl]propandinitril (Beispiel 14); 5-(3,3-Dicyanopropylsulfanyl)-3-ethylsulfanylpyridin-2-carbonsäuremethylester (Beispiel 15); 5-(3,3-Dicyanopropylsulfanyl)-3-ethylsulfanylpyridin-2-carbonsäureethylester (Beispiel 16); 3-Chlor-5-(3,3-dicyanopropylsulfanyl)pyridin-2-carbonsäureethylester (Beispiel 17); 5-(3,3-Dicyanopropylsulfanyl)-3-ethylsulfanylpyridin-2-carbonsäure (Beispiel 18); 3-Chlor-5-(3,3-dicyanopropylsulfanyl)pyridin-2-carbonsäure (Beispiel 19) und 5-(3,3-Dicyanopropylsulfanyl)-3-ethylsulfanylpyridin-2-carboxamid (Beispiel 20); Verbindung der Formel INT II nach Anspruch 15, ausgewählt aus 5-(3-Cyano-2-iminotetrahydrothiophen-3-yl)-3-ethylsulfanylpyridin-2-carbonsäure (Beispiel 21); oder Verbindung der Formel INT III nach Anspruch 16, ausgewählt aus 5-(1-Cyano-3-thiocyanatopropyl)-3-ethylsulfanylpyridin-2-carbonsäure (Beispiel 22).

19. Verbindung der Formel Ia, 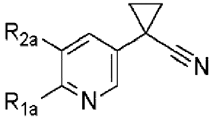 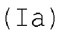
wobei R₁ₐ für -CO₂R₄ₐ, -CO(NR₅ₐR₆ₐ), Carboxylat oder Cyano steht; R₂ₐ für Wasserstoff, Halogen oder -SR₃ₐ steht; R₃ₐ für C₁-C₄-Alkyl oder C₃-C₆-Cycloalkyl-C₁-C₄-alkyl steht; R₄ₐ für Wasserstoff, -Si(CH₃)₃ oder C₁-C₆-Alkyl steht und R₅ₐ und R₆ₐ unabhängig voneinander für Wasserstoff oder C₁-C₄-Alkyl stehen, oder ein agrochemisch unbedenkliches Salz einer Verbindung der Formel Ia;
mit der Maßgabe, dass die Verbindung der Formel Ia nicht aus der Gruppe bestehend aus einer Verbindung der Formel Ib, Ic und Id ausgewählt ist; 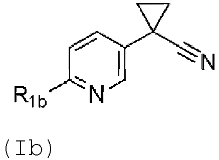
wobei R_{1b} für CN oder -CO₂Me steht; 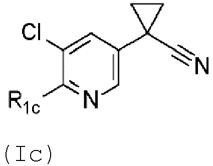
wobei R_{1c} für -CO₂H, -CO₂Me, -CO₂Et oder -CONHMe steht; und 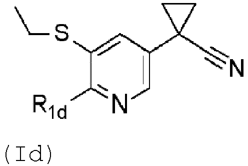
wobei R_{1d} für -CO₂H, -CO₂Me oder -CONHMe steht.

20. Verfahren zur Herstellung einer Verbindung der Formel I: 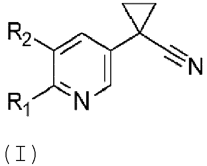 wobei R₁ für -CO₂R₄, -CO(NR₅R₆), Carboxylat oder Cyano steht; R₂ für Wasserstoff, Halogen oder -SR₃ steht; R₃ für C₁-C₄-Alkyl oder C₃-C₆-Cycloalkyl-C₁-C₄-alkyl steht; R₄ für Wasserstoff, -Si(CH₃)₃ oder C₁-C₆-Alkyl steht und R₅ und R₆ unabhängig voneinander für Wasserstoff oder C₁-C₄-Alkyl stehen, oder eines agrochemisch unbedenklichen Salzes einer Verbindung der Formel I; wobei das Verfahren Folgendes umfasst:
Umsetzen einer Verbindung der Formel (INT I), 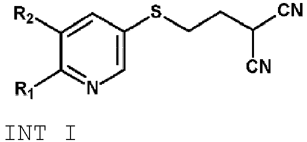
wobei R₁ und R₂ wie unter Formel I oben definiert sind, in Gegenwart einer geeigneten Base in einem passenden Lösungsmittel (oder Verdünnungsmittel) zur Herstellung einer Verbindung der Formel (I) oder eines agrochemisch unbedenklichen Salzes davon.

## Revendications

1. Procédé de préparation d'un composé de formule (I) : 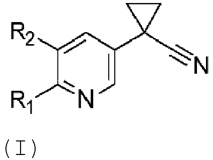 R₁ étant -CO₂R₄, -CO(NR₅R₆), carboxylate ou cyano ; R₂ étant hydrogène, halogène ou -SR₃ ; R₃ étant C₁-C₄alkyle ou C₃-C₆cycloalkyl-C₁-C₄alkyle ; R₄ étant hydrogène,-Si(CH₃)₃ ou C₁-C₆alkyle ; et R₅ et R₆ étant, indépendamment l'un de l'autre, hydrogène ou C₁-C₄alkyle, ou un sel acceptable sur le plan agrochimique d'un composé de formule I ; lequel procédé comprenant :
la mise en réaction d'un composé de formule (III) 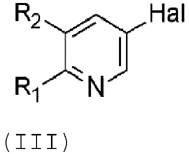
R₁ et R₂ étant tels que définis sous la formule I ci-dessus et Hal étant halogène
avec un composé de formule (IV), 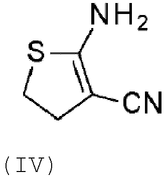
en la présence d'une base appropriée, dans un solvant (ou diluant) approprié ;
pour produire un composé de formule (I) ou un sel acceptable sur le plan agrochimique correspondant.

2. Procédé selon la revendication 1, R₁ étant -CO₂R₄,-CONH₂, carboxylate ou un groupe cyano ; et R₄ étant hydrogène, -Si(CH₃)₃, méthyle, éthyle, isopropyle, tert-butyle, ou sec-butyle.

3. Procédé selon la revendication 2, R₁ étant -CO₂H ou carboxylate.

4. Procédé selon l'une quelconque des revendications précédentes, R₂ étant hydrogène, chloro ou -SR₃ ;
R₃ étant éthyle.

5. Procédé selon la revendication 4, R₂ étant -S-éthyle.

6. Procédé selon l'une quelconque des revendications précédentes, Hal étant chloro ou bromo.

7. Procédé selon l'une quelconque des revendications précédentes, la base appropriée étant choisie parmi l'hydroxyde de sodium, l'hydroxyde de potassium, le tert-butanolate de sodium et le tert-butanolate de potassium.

8. Procédé selon l'une quelconque des revendications précédentes, le solvant (ou diluant) étant choisi parmi le diméthylformamide, le diméthylsulfoxyde, la N-méthyl-pyrrolidine, le diméthylacétamide, le sulfolane et la N,N'-diméthylpropylèneurée (DMPU).

9. Procédé selon l'une quelconque des revendications précédentes, qui est effectué dans une plage de température d'environ 0 °C à environ +100 °C, de préférence d'environ +20 °C à environ +80 °C.

10. Procédé selon l'une quelconque des revendications 1 - 6, 8 ou 9, lorsque R₁ est un ion carboxylate, la base appropriée étant choisie parmi le bis(tert-butanolate) de magnésium et le bis(hexaméthyldisilazoture) de magnésium.

11. Procédé selon l'une quelconque des revendications 1 - 6, 8 ou 9, lorsque R₁ est un anion carboxylate, la base appropriée étant choisie parmi l'hydroxyde de sodium, l'hydroxyde de potassium, le tert-butanolate de sodium et le tert-butanolate de potassium, et le procédé comprenant en outre l'ajout de sels de magnésium^{(II)}, zinc ^{(II)} ou Al^{(III)} comme additifs suite à la formation *in situ* d'un composé de formule (INT I) 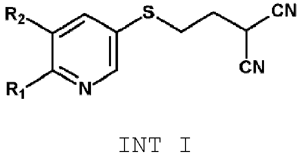 R₁ et R₂ étant tels que définis dans la formule I selon la revendication 1.

12. Procédé selon la revendication 11, les sels de magnésium^{(II)}, zinc ^{(II)} ou Al^{(III)} étant choisis parmi MgCl₂, ZnCl₂, Al(OtBu)₃ et AlCl₃.

13. Procédé selon l'une quelconque des revendications 1 - 6, 8 ou 9, lorsque R₁ est un anion carboxylate, la base appropriée étant choisie parmi l'hydroxyde de sodium, l'hydroxyde de potassium, le tert-butanolate de sodium et le tert-butanolate de potassium, et le procédé comprenant en outre l'ajout de chlorure de triméthylsilyle après distillation de l'eau ou de l'alcool généré(e) pendant la formation *in situ* d'un composé de formule (INT I) 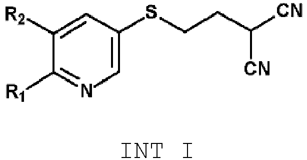 R₁ et R₂ étant tels que définis dans la formule I selon la revendication 1.

14. Composé de formule (INT I) 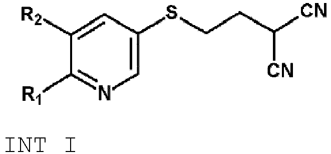 R₁ étant -CO₂R₄, -CO(NR₅R₆) ou cyano ; R₂ étant hydrogène, halogène ou -SR₃ ; R₃ étant C₁-C₄alkyle ou C₃-C₆cycloalkyl-C₁-C₄alkyle ; R₄ étant hydrogène, -Si(CH₃)₃ ou C₁-C₆alkyle ; et R₅ et R₆ étant, indépendamment l'un de l'autre, hydrogène ou C₁-C₄alkyle ; ou sel acceptable sur le plan agrochimique d'un composé de formule INT I.

15. Composé de formule (INT II) 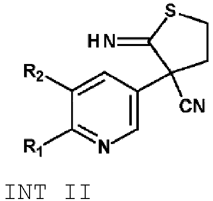 R₁ étant -CO₂R₄, -CO(NR₅R₆) ou cyano ; R₂ étant hydrogène, halogène ou -SR₃ ; R₃ étant C₁-C₄alkyle ou C₃-C₆cycloalkyl-C₁-C₄alkyle ; R₄ étant hydrogène, -Si (CH₃)₃ ou C₁-C₆alkyle ; et R₅ et R₆ étant, indépendamment l'un de l'autre, hydrogène ou C₁-C₄alkyle ; ou sel acceptable sur le plan agrochimique d'un composé de formule INT II.

16. Composé de formule (INT III) 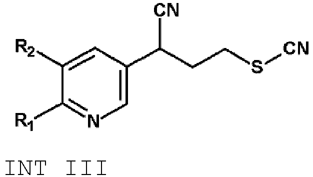 R₁ étant -CO₂R₄, -CO(NR₅R₆) ou cyano ; R₂ étant hydrogène, halogène ou -SR₃ ; R₃ étant C₁-C₄alkyle ou C₃-C₆cycloalkyl-C₁-C₄alkyle ; R₄ étant hydrogène, -Si (CH₃)₃ ou C₁-C₆alkyle ; et R₅ et R₆ étant, indépendamment l'un de l'autre, hydrogène ou C₁-C₄alkyle ; ou sel acceptable sur le plan agrochimique d'un composé de formule INT III.

17. Composé selon l'une quelconque des revendications 14, 15 ou 16, R₁ étant -CO₂R₄, -CONH₂ ou cyano ; R₄ étant hydrogène, -Si(CH₃)₃, méthyle, éthyle, isopropyle, tert-butyle, ou sec-butyle ; et R₂ étant hydrogène, chloro ou -SR₃ ; R₃ étant éthyle.

18. Composé de formule INT I selon la revendication 14, choisi dans le groupe constitué par :
2-[2-[(6-cyano-5-éthylsulfanyl-3-pyridyl)sulfanyl]éthyl]propanedinitrile (exemple 14) ; 5-(3,3-dicyanopropylsulfanyl)-3-éthylsulfanyl-pyridine-2-carboxylate de méthyle (exemple 15) ; 5-(3,3-dicyanopropylsulfanyl)-3-éthylsulfanyl-pyridine-2-carboxylate d'éthyle (exemple 16) ; 3-chloro-5-(3,3-dicyanopropylsulfanyl)pyridine-2-carboxylate d'éthyle (exemple 17) ; acide 5-(3,3-dicyanopropylsulfanyl)-3-éthylsulfanyl-pyridine-2-carboxylique (exemple 18) ; acide 3-chloro-5-(3,3-dicyanopropylsulfanyl)pyridine-2-carboxylique (exemple 19) ; et 5-(3,3-dicyanopropylsulfanyl)-3-éthylsulfanyl-pyridine-2-carboxamide (exemple 20) ; composé de formule INT II selon la revendication 15, choisi parmi acide 5-(3-cyano-2-imino-tétrahydrothiophène-3-yl)-3-éthylsulfanyl-pyridine-2-carboxylique (exemple 21) ; ou composé de formule INT III selon la revendication 16, choisi parmi acide 5-(1-cyano-3-thiocyanato-propyl)-3-éthylsulfanyl-pyridine-2-carboxylique (exemple 22).

19. Composé de formule la 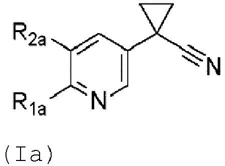
R₁ₐ étant -CO₂R₄ₐ, -CO(NR₅ₐR₆ₐ), carboxylate ou cyano ; R₂ₐ étant hydrogène, halogène ou -SR₃ₐ ; R₃ₐ étant C₁-C₄alkyle ou C₃-C₆cycloalkyl-C₁-C₄alkyle ; R₄ₐ étant hydrogène, - Si (CH₃)₃ ou C₁-C₆alkyle ; et R₅ₐ et R₆ₐ étant, indépendamment l'un de l'autre, hydrogène ou C₁-C₄alkyle, ou sel acceptable sur le plan agrochimique d'un composé de formule la ;
à la condition que le composé de formule la ne soit pas :
choisi dans le groupe constitué par un composé de la formule Ib, le et Id ; 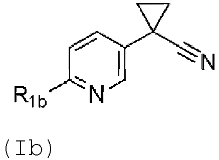
R_{1b} étant CN ou -CO₂Me ; 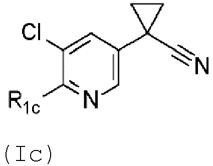
R_{1c} étant -CO₂H, -CO₂Me, -CO₂Et ou -CONHMe ; et 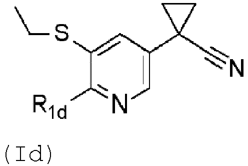
R_{1d} étant -CO₂H, -CO₂Me ou -CONHMe.

20. Procédé de préparation d'un composé de formule (I) : 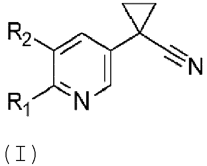 R₁ étant -CO₂R₄, -CO(NR₅R₆), carboxylate ou cyano ; R₂ étant hydrogène, halogène ou -SR₃ ; R₃ étant C₁-C₄alkyle ou C₃-C₆cycloalkyl-C₁-C₄alkyle ; R₄ étant hydrogène, - Si (CH₃)₃ ou C₁-C₆alkyle ; et R₅ et R₆ étant, indépendamment l'un de l'autre, hydrogène ou C₁-C₄alkyle, ou un sel acceptable sur le plan agrochimique d'un composé de formule I ; lequel procédé comprenant :
la mise en réaction d'un composé de formule (INT I) 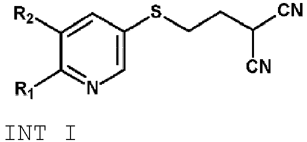
R₁ et R₂ étant tels que définis dans la formule I ci-dessus en la présence d'une base appropriée, dans un solvant (ou diluant) approprié; pour produire un composé de formule (I) ou un sel acceptable sur le plan agrochimique correspondant.
